# EUROPEAN PATENT APPLICATION

(11) **EP 4 006 057 A1**
(43) Date of publication of application: **01.06.2022**
(21) Application number: 20837897.6
(22) Date of filing: 10.07.2020
(51) Int. Cl.: C07K 19/00, A61K 47/50, A61K 38/00, C12N 15/11, C12N 15/63

(54) **COMPLEX FOR INTRACELLULAR DELIVERY OF MOLECULES**

(30) Priority: 11.07.2019 CN 201910624609
(71) Applicant: Xiamen University, Xiamen, Fujian 361005 (CN); Xiamen Innovax Biotech Co., Ltd., Xiamen, Fujian 361022 (CN)
(72) Inventor: GE, Shengxiang, Xiamen, Fujian 361005 (CN); YU, Siyuan, Xiamen, Fujian 361005 (CN); YANG, Han, Xiamen, Fujian 361005 (CN); PAN, Haifeng, Xiamen, Fujian 361005 (CN); REN, Shuling, Xiamen, Fujian 361005 (CN); LI, Tingdong, Xiamen, Fujian 361005 (CN); GUO, Qingshun, Xiamen, Fujian 361005 (CN); XIONG, Junhui, Xiamen, Fujian 361005 (CN); ZHANG, Jun, Xiamen, Fujian 361005 (CN); XIA, Ningshao, Xiamen, Fujian 361005 (CN)
(74) Representative: J A Kemp LLP
(86) International application number: PCT/CN2020/101424
(87) International publication number: WO 2021/004539

(57) **Abstract**

A fusion protein and a complex containing same, capable of being used for the intracellular delivery of cargo molecules. The fusion protein and complex can implement the efficient release of cargo molecules from endocytic vesicles, thereby significantly improving the cytoplasmic delivery efficiency of the cargo molecules. One cargo molecules can be obtained in cytoplasms, they can exert any function related thereto. The fusion protein and complex provide effective means for affecting biological mechanisms and pathways of cells, and can be used in various fields such as research, treatment, and diagnosis.

SEQUENCE LISTING

<110>  XIAMEN UNIVERSITY
       XIAMEN INNOVAX BIOTECH CO., LTD.

<120>  COMPLEX FOR INTRACELLULAR DELIVERY OF MOLECULES

<130>  N423544EP

<140>  EP20837897.6
<141>

<150>  CN201910624609.9
<151>  2019-07-11

<160>  54

<170>  PatentIn version 3.5

<210>  1
<211>  4
<212>  PRT
<213>  Artificial sequence

<220>
<223>  Furin recognition sequence-1


<220>
<221>  MISC_FEATURE
<222>  (2)..(2)
<223>  X=any amino acid

<220>
<221>  MISC_FEATURE
<222>  (3)..(3)
<223>  X=K or R

<400>  1

Arg Xaa Xaa Arg
1


<210>  2
<211>  5
<212>  PRT
<213>  Artificial sequence

<220>
<223>  Furin recognition sequence-2


<220>
<221>  MISC_FEATURE
<222>  (3)..(3)
<223>  X=any amino acid

<220>
<221>  MISC_FEATURE
<222>  (4)..(4)
<223>  X=K or R

<400>  2

Arg Arg Xaa Xaa Arg
1               5


<210>  3
<211>  5
<212>  PRT
<213>  Artificial sequence

<220>
<223>  Furin recognition sequence-3

<400>  3

Arg Arg His Lys Arg
1               5


<210>  4
<211>  15
<212>  PRT
<213>  Artificial sequence

<220>
<223>  Furin recognition sequence-4 (Ne)

<400>  4

Gln Ser Val Ala Ser Ser Arg Arg His Lys Arg Phe Ala Gly Val
1               5                   10                  15


<210>  5
<211>  45
<212>  DNA
<213>  Artificial sequence

<220>
<223>  Nucleic acid sequence encoding Ne

<400>  5
cagagcgttg caagcagccg tcgtcataaa cgttttgcag gtgtt                       45


<210>  6
<211>  15
<212>  PRT
<213>  Artificial sequence

<220>
<223>  CTSL recognition sequence N

<400>  6

Asn Asn Thr His Asp Leu Val Gly Asp Val Arg Leu Ala Gly Val
1               5                   10                  15


<210>  7
<211>  45
<212>  DNA

<213>  Artificial sequence

<220>
<223>  Nucleic acid sequence encoding N

<400>  7
aacaacactc atgaccttgt cggtgatgtg agattagccg gagtt                    45


<210>  8
<211>  23
<212>  PRT
<213>  Artificial sequence

<220>
<223>  INF7

<400>  8

Gly Leu Phe Glu Ala Ile Glu Gly Phe Ile Glu Asn Gly Trp Glu Gly
1               5                   10                  15


Met Ile Asp Gly Trp Tyr Gly
            20


<210>  9
<211>  69
<212>  DNA
<213>  Artificial sequence

<220>
<223>  Nucleic acid sequence encoding INF7

<400>  9
ggcctgttcg aagcaataga aggtttcata gaaaatggtt gggagggaat gatagacggt      60

tggtacggt                                                              69


<210>  10
<211>  13
<212>  PRT
<213>  Artificial sequence

<220>
<223>  Tat(48-60)

<400>  10

Gly Arg Lys Lys Arg Arg Gln Arg Arg Arg Pro Pro Gln
1               5                   10


<210>  11
<211>  39
<212>  DNA
<213>  Artificial sequence

<220>
<223>  Nucleic acid sequence encoding Tat (48-60)

<400>  11
ggccgtaaga agcggagaca gcgacgaaga ccgccgcag                                39


<210>  12
<211>  56
<212>  PRT
<213>  Artificial sequence

<220>
<223>  Fusion protein TIN

<400>  12

Gly Arg Lys Lys Arg Arg Gln Arg Arg Arg Pro Pro Gln Gly Leu Phe
1               5                   10                  15


Glu Ala Ile Glu Gly Phe Ile Glu Asn Gly Trp Glu Gly Met Ile Asp
            20                  25                  30


Gly Trp Tyr Gly Gly Gly Gly Gly Ser Asn Asn Thr His Asp Leu Val
        35                  40                  45


Gly Asp Val Arg Leu Ala Gly Val
    50                  55


<210>  13
<211>  56
<212>  PRT
<213>  Artificial sequence

<220>
<223>  Fusion protein TINe

<400>  13

Gly Arg Lys Lys Arg Arg Gln Arg Arg Arg Pro Pro Gln Gly Leu Phe
1               5                   10                  15


Glu Ala Ile Glu Gly Phe Ile Glu Asn Gly Trp Glu Gly Met Ile Asp
            20                  25                  30


Gly Trp Tyr Gly Gly Gly Gly Gly Ser Gln Ser Val Ala Ser Ser Arg
        35                  40                  45


Arg His Lys Arg Phe Ala Gly Val
    50                  55


<210>  14
<211>  76
<212>  PRT
<213>  Artificial sequence

<220>
<223>  Fusion protein TINNe

<400>  14

Gly Arg Lys Lys Arg Arg Gln Arg Arg Arg Pro Pro Gln Gly Leu Phe
1               5                   10                  15

Glu Ala Ile Glu Gly Phe Ile Glu Asn Gly Trp Glu Gly Met Ile Asp
            20                  25                  30

Gly Trp Tyr Gly Gly Gly Gly Gly Ser Asn Asn Thr His Asp Leu Val
        35                  40                  45

Gly Asp Val Arg Leu Ala Gly Val Gly Gly Gly Gly Ser Gln Ser Val
    50                  55                  60

Ala Ser Ser Arg Arg His Lys Arg Phe Ala Gly Val
65                  70                  75

<210>  15
<211>  7
<212>  PRT
<213>  Artificial sequence

<220>
<223>  NLS

<400>  15

Pro Lys Lys Lys Arg Lys Val
1               5

<210>  16
<211>  21
<212>  PRT
<213>  Artificial sequence

<220>
<223>  CTSL recognition sequence Na

<400>  16

Ala Arg Ala Tyr Gly Phe Arg Gly Pro Gly Pro Gln Leu Arg Arg Gly
1               5                   10                  15

Trp Arg Pro Ser Ser
            20

<210>  17
<211>  24
<212>  PRT
<213>  Artificial sequence

<220>
<223>  CTSL recognition sequence Nb

<400>  17

Glu Glu Asp Asn Arg Asp Ser Ser Met Lys Leu Ser Phe Arg Ala Arg
1               5                   10                  15

Ala Tyr Gly Phe Arg Gly Pro Gly
            20

<210>  18
<211>  17
<212>  PRT
<213>  Artificial sequence

<220>
<223>  Furin recognition sequence Nc

<400>  18

Gly Thr Ser Gly Gly Arg Gln Arg Arg Glu Leu Asp Pro Met Asp Ile
1               5                   10                  15

Arg

<210>  19
<211>  20
<212>  PRT
<213>  Artificial sequence

<220>
<223>  Furin recognition sequence Nd

<400>  19

Glu Asp Thr Asp Phe Ala Arg Lys Asn Lys Ile Leu Tyr Thr Gln Gln
1               5                   10                  15

Val His Gln Thr
            20

<210>  20
<211>  9
<212>  PRT
<213>  Artificial sequence

<220>
<223>  CTSD identification sequence Nf

<400>  20

Lys Pro Ile Leu Phe Phe Arg Leu Lys
1               5

<210>  21
<211>  15
<212>  PRT

<213>  Artificial sequence

<220>
<223>  Mutant N

<400>  21

Asn Asn Thr His Asp Leu Val Gly Asp Val Gly Leu Ala Gly Val
1               5                   10                  15


<210>  22
<211>  15
<212>  PRT
<213>  Artificial sequence

<220>
<223>  Mutant Ne

<400>  22

Gln Ser Val Ala Ser Ser Arg Arg His Lys Gly Phe Ala Gly Val
1               5                   10                  15


<210>  23
<211>  228
<212>  PRT
<213>  Artificial sequence

<220>
<223>  GFP(beta)1-10-NLS

<400>  23

Met Gly Ser Lys Gly Glu Glu Leu Phe Thr Gly Val Val Pro Ile Leu
1               5                   10                  15


Val Glu Leu Asp Gly Asp Val Asn Gly His Lys Phe Ser Val Arg Gly
            20                  25                  30


Glu Gly Glu Gly Asp Ala Thr Ile Gly Lys Leu Thr Leu Lys Phe Ile
        35                  40                  45


Cys Thr Thr Gly Lys Leu Pro Val Pro Trp Pro Thr Leu Val Thr Thr
    50                  55                  60


Leu Thr Tyr Gly Val Gln Cys Phe Ser Arg Tyr Pro Asp His Met Lys
65                  70                  75                  80


Arg His Asp Phe Phe Lys Ser Ala Met Pro Glu Gly Tyr Val Gln Glu
                85                  90                  95


Arg Thr Ile Ser Phe Lys Asp Asp Gly Lys Tyr Lys Thr Arg Ala Val
            100                 105                 110

Val Lys Phe Glu Gly Asp Thr Leu Val Asn Arg Ile Glu Leu Lys Gly
        115                 120                 125

Thr Asp Phe Lys Glu Asp Gly Asn Ile Leu Gly His Lys Leu Glu Tyr
    130                 135                 140

Asn Phe Asn Ser His Asn Val Tyr Ile Thr Ala Asp Lys Gln Lys Asn
145                 150                 155                 160

Gly Ile Lys Ala Asn Phe Thr Val Arg His Asn Val Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Thr Val Leu
        195                 200                 205

Ser Lys Asp Pro Asn Glu Lys His His His His His His Pro Lys Lys
    210                 215                 220

Lys Arg Lys Val
225

<210>  24
<211>  687
<212>  DNA
<213>  Artificial sequence

<220>
<223>  Nucleic acid sequence encoding GFP(beta)1-10-NLS

<400>  24
atgggcagca aaggagaaga acttttcact ggagttgtcc caattcttgt tgaattagat      60

ggtgatgtta atgggcacaa attttctgtc agaggagagg gtgaaggtga tgctacaatc     120

ggaaaactca cccttaaatt tatttgcact actggaaaac tacctgttcc atggccaaca     180

cttgtcacta ctctgaccta tggtgttcaa tgcttttccc gttatccgga tcacatgaaa     240

aggcatgact ttttcaagag tgccatgccc gaaggttatg tacaggaacg cactatatct     300

ttcaaagatg acgggaaata caagacgcgt gctgtagtca agtttgaagg tgataccctt     360

gttaatcgta tcgagttaaa gggtactgat tttaaagaag atggaaacat tctcggacac     420

aaactcgagt acaactttaa ctcacacaat gtatacatca cggcagacaa acaaaagaat     480

ggaatcaaag ctaacttcac agttcgccac aacgttgaag atggttccgt tcaactagca     540

gaccattatc aacaaaatac tccaattggc gatggccctg tccttttacc agacaaccat     600

tacctgtcga cacaaactgt cctttcgaaa gatcccaacg aaaagcacca ccaccaccac     660

caccccaaga agaagaggaa ggtgtaa                                          687

<210>  25
<211>  411
<212>  DNA
<213>  Artificial sequence

<220>
<223>  Nucleic acid sequence encoding Histone-H3

<400>  25
atggctcgca ctaagcaaac tgctcggaag tctactggtg gcaaggcgcc acgcaaacag      60

ttggccacta aggcagcccg caaaagcgct ccggccaccg gcggcgtgaa aaagccccac     120

cgctaccggc cgggcaccgt ggctctgcgc gagatccgcc gttatcagaa gtccactgaa     180

ctgcttattc gtaaactacc tttccagcgc ctggtgcgcg agattgcgca ggactttaaa     240

acagacctgc gtttccagag ctccgctgtg atggctctgc aggaggcgtg cgaggcctac     300

ttggtagggc tatttgagga cactaacctg tgcgccatcc acgccaagcg cgtcactatc     360

atgcccaagg acatccagct cgcccgccgc atccgcggag agagggcgtg a              411

<210>  26
<211>  47
<212>  DNA
<213>  Artificial sequence

<220>
<223>  Nucleic acid sequence encoding GFP(beta)11

<400>  26
cgggaccaca tggtgctgca cgagtacgtg aacgccgccg gcatcac                    47

<210>  27
<211>  98
<212>  PRT
<213>  Artificial sequence

<220>
<223>  ZFP9-NLS

<400>  27

Val Ser Arg Pro Gly Glu Arg Pro Phe Gln Cys Arg Ile Cys Met Arg
1               5                   10                  15

Asn Phe Ser Asp Lys Thr Lys Leu Arg Val His Thr Arg Thr His Thr
            20                  25                  30

Gly Glu Lys Pro Phe Gln Cys Arg Ile Cys Met Arg Asn Phe Ser Val
        35                  40                  45

Arg His Asn Leu Thr Arg His Leu Arg Thr His Thr Gly Glu Lys Pro
    50                  55                  60

Phe Gln Cys Arg Ile Cys Met Arg Asn Phe Ser Gln Ser Thr Ser Leu
65                  70                  75                  80

Gln Arg His Leu Lys Thr His Leu Arg Gly Ser Pro Lys Lys Lys Arg
                85                  90                  95

Lys Val

<210>  28
<211>  294
<212>  DNA
<213>  Artificial sequence

<220>
<223>  Nucleic acid sequence encoding ZFP9-NLS

<400>  28
gtctctagac ccggggagcg ccccttccag tgtcgcattt gcatgcggaa cttttcggat      60

aaaactaaat tgagagttca tacccgtact cataccggtg aaaaaccgtt tcagtgtcgg     120

atctgtatgc gaaatttctc cgttagacat aatttgacta gacatctacg tacgcacacc     180

ggcgagaagc cattccaatg ccgaatatgc atgcgcaact tcagtcaatc tacttctttg     240

caaagacacc taaaaaccca cctgagagga tcccccaaga agaagcgtaa ggtg           294

<210>  29
<211>  714
<212>  DNA
<213>  Artificial sequence

<220>
<223>  Sequence encoding BFP

<400>  29
atggtgtcta agggcgaaga gctgattaag gagaacatgc acatgaagct gtacatggag      60

ggcaccgtgg acaaccatca cttcaagtgc acatccgagg gcgaaggcaa gccctacgag     120

ggcacccaga ccatgagaat caaggtggtc gagggcggcc ctctcccctt cgccttcgac     180

atcctggcta ctagcttcct ctacggcagc aagaccttca tcaaccacac ccagggcatc     240

cccgacttct tcaagcagtc cttccctgag ggcttcacat gggagagagt caccacatac     300

gaagacgggg gcgtgctgac cgctacccag gacaccagcc tccaggacgg ctgcctcatc     360

tacaacgtca agatcagagg ggtgaacttc acatccaacg gccctgtgat gcagaagaaa     420

acactcggct gggaggcctt caccgagacg ctgtaccccg ctgacggcgg cctggaaggc     480

agaaacgaca tggccctgaa gctcgtgggc gggagccatc tgatcgcaaa cgccaagacc     540

acatatagat ccaagaaacc cgctaagaac ctcaagatgc ctggcgtcta ctatgtggac     600

tacagactgg aaagaatcaa ggaggccaac aacgagacct acgtcgagca gcacgaggtg 660

gcagtggcca gatactgcga cctccctagc aaactggggc acaagcttaa ttga 714

<210> 30
<211> 11
<212> DNA
<213> Artificial sequence

<220>
<223> ZFP9 binding site sequence

<400> 30
tgtagatgga g 11

<210> 31
<211> 378
<212> PRT
<213> Artificial sequence

<220>
<223> Ppm1b

<400> 31

Met Gly Ala Phe Leu Asp Lys Pro Lys Thr Glu Lys His Asn Ala His
1               5                   10                  15

Gly Ala Gly Asn Gly Leu Arg Tyr Gly Leu Ser Ser Met Gln Gly Trp
            20                  25                  30

Arg Val Glu Met Glu Asp Ala His Thr Ala Val Val Gly Ile Pro His
        35                  40                  45

Gly Leu Asp Asn Trp Ser Phe Phe Ala Val Tyr Asp Gly His Ala Gly
    50                  55                  60

Ser Arg Val Ala Asn Tyr Cys Ser Thr His Leu Leu Glu His Ile Thr
65                  70                  75                  80

Thr Asn Glu Asp Phe Arg Ala Ala Asp Lys Ser Gly Ser Ala Leu Glu
                85                  90                  95

Pro Ser Val Glu Ser Val Lys Thr Gly Ile Arg Thr Gly Phe Leu Lys
            100                 105                 110

Ile Asp Glu Tyr Met Arg Asn Phe Ser Asp Leu Arg Asn Gly Met Asp
        115                 120                 125

Arg Ser Gly Ser Thr Ala Val Gly Val Met Val Ser Pro Thr His Met
    130                 135                 140

Tyr Phe Ile Asn Cys Gly Asp Ser Arg Ala Val Leu Cys Arg Asn Gly
145                 150                 155                 160

Gln Val Cys Phe Ser Thr Gln Asp His Lys Pro Cys Asn Pro Val Glu
                165                 170                 175

Lys Glu Arg Ile Gln Asn Ala Gly Gly Ser Val Met Ile Gln Arg Val
            180                 185                 190

Asn Gly Ser Leu Ala Val Ser Arg Ala Leu Gly Asp Tyr Asp Tyr Lys
        195                 200                 205

Cys Val Asp Gly Lys Gly Pro Thr Glu Gln Leu Val Ser Pro Glu Pro
    210                 215                 220

Glu Val Tyr Glu Ile Val Arg Ala Glu Glu Asp Glu Phe Val Val Leu
225                 230                 235                 240

Ala Cys Asp Gly Ile Trp Asp Val Met Ser Asn Glu Glu Leu Cys Glu
                245                 250                 255

Phe Val Lys Ser Arg Leu Glu Val Ser Asp Asp Leu Glu Asn Val Cys
            260                 265                 270

Asn Trp Val Val Asp Thr Cys Leu His Lys Gly Ser Arg Asp Asn Met
        275                 280                 285

Ser Val Val Leu Val Cys Phe Ser Asn Ala Pro Lys Val Ser Glu Glu
    290                 295                 300

Ala Val Lys Arg Asp Ser Glu Leu Asp Lys His Leu Glu Ser Arg Val
305                 310                 315                 320

Glu Glu Ile Met Gln Lys Ser Gly Glu Glu Gly Met Pro Asp Leu Ala
                325                 330                 335

His Val Met Arg Ile Leu Ser Ala Glu Asn Ile Pro Asn Leu Pro Pro
            340                 345                 350

Gly Gly Gly Leu Ala Gly Lys Arg His Val Ile Glu Ala Val Tyr Ser
        355                 360                 365

Arg Leu Asn Pro His Lys Asp Asn Asp Gly
    370                 375

<210>  32
<211>  1134
<212>  DNA

<213>  Artificial sequence

<220>
<223>  Nucleic acid sequence encoding Ppm1b

<400>  32
atgggtgcat ttttggataa acccaaaact gaaaagcaca atgctcacgg tgctgggaat      60

ggtctgcgtt atggcctgag cagtatgcaa ggatggagag tagaaatgga agatgcacac     120

acagctgttg tgggtattcc tcacggcttg gacaactggt cgttttttgc agtttatgac     180

ggtcatgctg gatcccgagt ggcaaattac tgttcaacac atctattaga acacatcacc     240

accaatgaag acttcagggc agctgacaaa tcaggctctg ctctcgagcc ttcagtagaa     300

agtgtgaaga ctggtatcag gactggcttt ttgaaaattg atgaatatat gcgtaacttc     360

tcagacctga ggaacgggat ggacaggagc ggctcgactg cagtgggcgt gatggtctcc     420

cctacacaca tgtacttcat caactgtggt gactctcgag ctgttctgtg taggaacgga     480

caggtctgct tttctaccca ggatcacaaa ccttgtaatc cagtggagaa ggagcgcatc     540

caaaacgcag gaggcagtgt gatgatccag cgtgtgaacg gctcactagc agtgtctcgg     600

gctctggggg actatgacta caagtgtgtg gatggcaagg gccctacaga gcagcttgtt     660

tctccagagc ctgaggttta tgagattgtg agagcagaag aggatgagtt tgtcgtcttg     720

gcctgtgatg ggatctggga tgtgatgagc aatgaggagc tctgtgagtt tgttaagtct     780

aggcttgagg tgtcggacga cctggagaat gtgtgcaatt gggtagtgga cacttgttta     840

cataagggaa gtcgagataa catgagtgtt gtattagttt gcttttcaaa tgcccccaag     900

gtctcagagg aagccgtgaa gagagattca gagttggata agcacttgga atcacgggtt     960

gaagaaatca tgcagaagtc tggggaggaa ggaatgcctg atcttgccca tgtgatgcgc    1020

attttgtctg cagaaaatat cccgaattta cctcccgggg gaggtctcgc tggcaagcgc    1080

catgttattg aagctgttta tagtagactt aatccacaca aagacaatga tggg          1134

<210>  33
<211>  1368
<212>  PRT
<213>  Artificial sequence

<220>
<223>  Cas9-NLS

<400>  33

Met Asp Lys Lys Tyr Ser Ile Gly Leu Asp Ile Gly Thr Asn Ser Val
1               5                   10                  15

Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
        35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
        115                 120                 125

His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
            180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
                405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
                485                 490                 495

Asn Phe Asp Lys Asn Leu Pro Asn Glu Lys Val Leu Pro Lys His Ser
            500                 505                 510

Leu Leu Tyr Glu Tyr Phe Thr Val Tyr Asn Glu Leu Thr Lys Val Lys
        515                 520                 525

Tyr Val Thr Glu Gly Met Arg Lys Pro Ala Phe Leu Ser Gly Glu Gln
    530                 535                 540

Lys Lys Ala Ile Val Asp Leu Leu Phe Lys Thr Asn Arg Lys Val Thr
545                 550                 555                 560

Val Lys Gln Leu Lys Glu Asp Tyr Phe Lys Lys Ile Glu Cys Phe Asp
                565                 570                 575

Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605

Asn Glu Glu Asn Glu Asp Ile Leu Glu Asp Ile Val Leu Thr Leu Thr
    610                 615                 620

Leu Phe Glu Asp Arg Glu Met Ile Glu Glu Arg Leu Lys Thr Tyr Ala
625                 630                 635                 640

His Leu Phe Asp Asp Lys Val Met Lys Gln Leu Lys Arg Arg Arg Tyr
                645                 650                 655

Thr Gly Trp Gly Arg Leu Ser Arg Lys Leu Ile Asn Gly Ile Arg Asp
            660                 665                 670

Lys Gln Ser Gly Lys Thr Ile Leu Asp Phe Leu Lys Ser Asp Gly Phe
        675                 680                 685

Ala Asn Arg Asn Phe Met Gln Leu Ile His Asp Asp Ser Leu Thr Phe
    690                 695                 700

Lys Glu Asp Ile Gln Lys Ala Gln Val Ser Gly Gln Gly Asp Ser Leu
705                 710                 715                 720

His Glu His Ile Ala Asn Leu Ala Gly Ser Pro Ala Ile Lys Lys Gly
                725                 730                 735

Ile Leu Gln Thr Val Lys Val Val Asp Glu Leu Val Lys Val Met Gly
            740                 745                 750

Arg His Lys Pro Glu Asn Ile Val Ile Glu Met Ala Arg Glu Asn Gln
        755                 760                 765

Thr Thr Gln Lys Gly Gln Lys Asn Ser Arg Glu Arg Met Lys Arg Ile
    770                 775                 780

Glu Glu Gly Ile Lys Glu Leu Gly Ser Gln Ile Leu Lys Glu His Pro

785                 790                 795                 800

Val Glu Asn Thr Gln Leu Gln Asn Glu Lys Leu Tyr Leu Tyr Tyr Leu
                805                 810                 815

Gln Asn Gly Arg Asp Met Tyr Val Asp Gln Glu Leu Asp Ile Asn Arg
            820                 825                 830

Leu Ser Asp Tyr Asp Val Asp Ala Ile Val Pro Gln Ser Phe Leu Lys
        835                 840                 845

Asp Asp Ser Ile Asp Asn Lys Val Leu Thr Arg Ser Asp Lys Asn Arg
    850                 855                 860

Gly Lys Ser Asp Asn Val Pro Ser Glu Glu Val Val Lys Lys Met Lys
865                 870                 875                 880

Asn Tyr Trp Arg Gln Leu Leu Asn Ala Lys Leu Ile Thr Gln Arg Lys
                885                 890                 895

Phe Asp Asn Leu Thr Lys Ala Glu Arg Gly Gly Leu Ser Glu Leu Asp
            900                 905                 910

Lys Ala Gly Phe Ile Lys Arg Gln Leu Val Glu Thr Arg Gln Ile Thr
        915                 920                 925

Lys His Val Ala Gln Ile Leu Asp Ser Arg Met Asn Thr Lys Tyr Asp
    930                 935                 940

Glu Asn Asp Lys Leu Ile Arg Glu Val Lys Val Ile Thr Leu Lys Ser
945                 950                 955                 960

Lys Leu Val Ser Asp Phe Arg Lys Asp Phe Gln Phe Tyr Lys Val Arg
                965                 970                 975

Glu Ile Asn Asn Tyr His His Ala His Asp Ala Tyr Leu Asn Ala Val
            980                 985                 990

Val Gly Thr Ala Leu Ile Lys Lys  Tyr Pro Lys Leu Glu  Ser Glu Phe
        995                 1000                1005

Val Tyr  Gly Asp Tyr Lys Val  Tyr Asp Val Arg Lys  Met Ile Ala
    1010                1015                1020

Lys Ser  Glu Gln Glu Ile Gly  Lys Ala Thr Ala Lys  Tyr Phe Phe
    1025                1030                1035

Tyr Ser Asn Ile Met Asn Phe Phe Lys Thr Glu Ile Thr Leu Ala
    1040                1045                1050

Asn Gly Glu Ile Arg Lys Arg Pro Leu Ile Glu Thr Asn Gly Glu
    1055                1060                1065

Thr Gly Glu Ile Val Trp Asp Lys Gly Arg Asp Phe Ala Thr Val
    1070                1075                1080

Arg Lys Val Leu Ser Met Pro Gln Val Asn Ile Val Lys Lys Thr
    1085                1090                1095

Glu Val Gln Thr Gly Gly Phe Ser Lys Glu Ser Ile Leu Pro Lys
    1100                1105                1110

Arg Asn Ser Asp Lys Leu Ile Ala Arg Lys Lys Asp Trp Asp Pro
    1115                1120                1125

Lys Lys Tyr Gly Gly Phe Asp Ser Pro Thr Val Ala Tyr Ser Val
    1130                1135                1140

Leu Val Val Ala Lys Val Glu Lys Gly Lys Ser Lys Lys Leu Lys
    1145                1150                1155

Ser Val Lys Glu Leu Leu Gly Ile Thr Ile Met Glu Arg Ser Ser
    1160                1165                1170

Phe Glu Lys Asn Pro Ile Asp Phe Leu Glu Ala Lys Gly Tyr Lys
    1175                1180                1185

Glu Val Lys Lys Asp Leu Ile Ile Lys Leu Pro Lys Tyr Ser Leu
    1190                1195                1200

Phe Glu Leu Glu Asn Gly Arg Lys Arg Met Leu Ala Ser Ala Gly
    1205                1210                1215

Glu Leu Gln Lys Gly Asn Glu Leu Ala Leu Pro Ser Lys Tyr Val
    1220                1225                1230

Asn Phe Leu Tyr Leu Ala Ser His Tyr Glu Lys Leu Lys Gly Ser
    1235                1240                1245

Pro Glu Asp Asn Glu Gln Lys Gln Leu Phe Val Glu Gln His Lys
    1250                1255                1260

His Tyr Leu Asp Glu Ile Ile Glu Gln Ile Ser Glu Phe Ser Lys
    1265                1270                1275

Arg Val Ile Leu Ala Asp Ala Asn Leu Asp Lys Val Leu Ser Ala
    1280                1285                1290

Tyr Asn Lys His Arg Asp Lys Pro Ile Arg Glu Gln Ala Glu Asn
    1295                1300                1305

Ile Ile His Leu Phe Thr Leu Thr Asn Leu Gly Ala Pro Ala Ala
    1310                1315                1320

Phe Lys Tyr Phe Asp Thr Thr Ile Asp Arg Lys Arg Tyr Thr Ser
    1325                1330                1335

Thr Lys Glu Val Leu Asp Ala Thr Leu Ile His Gln Ser Ile Thr
    1340                1345                1350

Gly Leu Tyr Glu Thr Arg Ile Asp Leu Ser Gln Leu Gly Gly Asp
    1355                1360                1365

<210>  34
<211>  4125
<212>  DNA
<213>  Artificial sequence

<220>
<223>  Nucleic acid sequence encoding Cas9-NLS

<400>  34
atggataaga aatactcaat aggcttagat atcggcacaa atagcgtcgg atgggcggtg      60

atcactgatg aatataaggt tccgtctaaa aagttcaagg ttctgggaaa tacagaccgc     120

cacagtatca aaaaaaatct tataggggct cttttatttg acagtggaga gacagcggaa     180

gcgactcgtc tcaaacggac agctcgtaga aggtatacac gtcggaagaa tcgtatttgt     240

tatctacagg agattttttc aaatgagatg gcgaaagtag atgatagttt ctttcatcga     300

cttgaagagt cttttttggt ggaagaagac aagaagcatg aacgtcatcc tatttttgga     360

aatatagtag atgaagttgc ttatcatgag aaatatccaa ctatctatca tctgcgaaaa     420

aaattggtag attctactga taaagcggat ttgcgcttaa tctatttggc cttagcgcat     480

atgattaagt ttcgtggtca ttttttgatt gagggagatt taaatcctga taatagtgat     540

gtggacaaac tatttatcca gttggtacaa acctacaatc aattatttga agaaaaccct     600

attaacgcaa gtggagtaga tgctaaagcg attctttctg cacgattgag taaatcaaga     660

cgattagaaa atctcattgc tcagctcccc ggtgagaaga aaaatggctt atttgggaat     720

ctcattgctt tgtcattggg tttgacccct aattttaaat caaattttga tttggcagaa     780

gatgctaaat tacagctttc aaaagatact tacgatgatg atttagataa tttattggcg     840

caaattggag atcaatatgc tgatttgttt ttggcagcta agaatttatc agatgctatt     900

ttactttcag atatcctaag agtaaatact gaaataacta aggctcccct atcagcttca 960

atgattaaac gctacgatga acatcatcaa gacttgactc ttttaaaagc tttagttcga 1020

caacaacttc cagaaaagta taaagaaatc ttttttgatc aatcaaaaaa cggatatgca 1080

ggttatattg atgggggagc tagccaagaa gaattttata aatttatcaa accaatttta 1140

gaaaaaatgg atggtactga ggaattattg gtgaaactaa atcgtgaaga tttgctgcgc 1200

aagcaacgga cctttgacaa cggctctatt ccccatcaaa ttcacttggg tgagctgcat 1260

gctattttga gaagacaaga agacttttat ccatttttaa aagacaatcg tgagaagatt 1320

gaaaaaatct tgacttttcg aattccttat tatgttggtc cattggcgcg tggcaatagt 1380

cgttttgcat ggatgactcg gaagtctgaa gaaacaatta ccccatggaa ttttgaagaa 1440

gttgtcgata aaggtgcttc agctcaatca tttattgaac gcatgacaaa ctttgataaa 1500

aatcttccaa atgaaaaagt actaccaaaa catagtttgc tttatgagta ttttacggtt 1560

tataacgaat tgacaaaggt caaatatgtt actgaaggaa tgcgaaaacc agcatttctt 1620

tcaggtgaac agaagaaagc cattgttgat ttactcttca aaacaaatcg aaaagtaacc 1680

gttaagcaat taaaagaaga ttatttcaaa aaaatagaat gttttgatag tgttgaaatt 1740

tcaggagttg aagatagatt taatgcttca ttaggtacct accatgattt gctaaaaatt 1800

attaaagata aagatttttt ggataatgaa gaaaatgaag atatcttaga ggatattgtt 1860

ttaacattga ccttatttga agatagggag atgattgagg aaagacttaa aacatatgct 1920

cacctctttg atgataaggt gatgaaacag cttaaacgtc gccgttatac tggttgggga 1980

cgtttgtctc gaaaattgat taatggtatt agggataagc aatctggcaa aacaatatta 2040

gattttttga aatcagatgg ttttgccaat cgcaatttta tgcagctgat ccatgatgat 2100

agtttgacat ttaaagaaga cattcaaaaa gcacaagtgt ctggacaagg cgatagttta 2160

catgaacata ttgcaaattt agctggtagc cctgctatta aaaaaggtat tttacagact 2220

gtaaaagttg ttgatgaatt ggtcaaagta atggggcggc ataagccaga aaatatcgtt 2280

attgaaatgg cacgtgaaaa tcagacaact caaaagggcc agaaaaattc gcgagagcgt 2340

atgaaacgaa tcgaagaagg tatcaaagaa ttaggaagtc agattcttaa agagcatcct 2400

gttgaaaata ctcaattgca aaatgaaaag ctctatctct attatctcca aaatggaaga 2460

gacatgtatg tggaccaaga attagatatt aatcgtttaa gtgattatga tgtcgatcac 2520

attgttccac aaagtttcct taaagacgat tcaatagaca ataaggtctt aacgcgttct 2580

gataaaaatc gtggtaaatc ggataacgtt ccaagtgaag aagtagtcaa aaagatgaaa 2640

aactattgga gacaacttct aaacgccaag ttaatcactc aacgtaagtt tgataattta 2700

acgaaagctg aacgtggagg tttgagtgaa cttgataaag ctggttttat caaacgccaa 2760

ttggttgaaa ctcgccaaat cactaagcat gtggcacaaa ttttggatag tcgcatgaat 2820

actaaatacg atgaaaatga taaacttatt cgagaggtta aagtgattac cttaaaatct 2880

aaattagttt ctgacttccg aaaagatttc caattctata aagtacgtga gattaacaat 2940

taccatcatg cccatgatgc gtatctaaat gccgtcgttg gaactgcttt gattaagaaa 3000

tatccaaaac ttgaatcgga gtttgtctat ggtgattata aagtttatga tgttcgtaaa 3060

atgattgcta agtctgagca agaaataggc aaagcaaccg caaaatattt cttttactct 3120

aatatcatga acttcttcaa aacagaaatt acacttgcaa atggagagat tcgcaaacgc 3180

cctctaatcg aaactaatgg ggaaactgga gaaattgtct gggataaagg gcgagatttt 3240

gccacagtgc gcaaagtatt gtccatgccc caagtcaata ttgtcaagaa aacagaagta 3300

cagacaggcg gattctccaa ggagtcaatt ttaccaaaaa gaaattcgga caagcttatt 3360

gctcgtaaaa aagactggga tccaaaaaaa tatggtggtt ttgatagtcc aacggtagct 3420

tattcagtcc tagtggttgc taaggtggaa aaagggaaat cgaagaagtt aaaatccgtt 3480

aaagagttac tagggatcac aattatggaa agaagttcct ttgaaaaaaa tccgattgac 3540

tttttagaag ctaaaggata taaggaagtt aaaaaagact taatcattaa actacctaaa 3600

tatagtcttt ttgagttaga aaacggtcgt aaacggatgc tggctagtgc cggagaatta 3660

caaaaaggaa atgagctggc tctgccaagc aaatatgtga attttttata tttagctagt 3720

cattatgaaa agttgaaggg tagtccagaa gataacgaac aaaaacaatt gtttgtggag 3780

cagcataagc attatttaga tgagattatt gagcaaatca gtgaattttc taagcgtgtt 3840

attttagcag atgccaattt agataaagtt cttagtgcat ataacaaaca tagagacaaa 3900

ccaatacgtg aacaagcaga aaatattatt catttattta cgttgacgaa tcttggagct 3960

cccgctgctt ttaaatattt tgatacaaca attgatcgta aacgatatac gtctacaaaa 4020

gaagttttag atgccactct tatccatcaa tccatcactg gtctttatga aacacgcatt 4080

gatttgagtc agctaggagg tgaccccaag aagaagagga aggtg 4125

<210> 35
<211> 672
<212> DNA
<213> Artificial sequence

<220>
<223> Nucleic acid sequence encoding dsRed

<400> 35
gcctcctccg agaacgtcat caccgagttc atgcgcttca aggtgcgcat ggagggcacc 60

gtgaacggcc acgagttcga gatcgagggc gagggcgagg gccgccccta cgagggccac 120

aacaccgtga agctgaaggt gaccaagggc ggccccctgc ccttcgcctg ggacatcctg 180

tccccccagt tccagtacgg ctccaaggtg tacgtgaagc accccgccga catccccgac 240

tacaagaagc tgtccttccc cgagggcttc aagtgggagc gcgtgatgaa cttcgaggac 300

ggcggcgtgg cgaccgtgac ccaggactcc tccctgcagg acggctgctt catctacaag 360

gtgaagttca tcggcgtgaa cttcccctcc gacggccccg tgatgcagaa gaagaccatg 420

ggctgggagg cctccaccga gcgcctgtac ccccgcgacg gcgtgctgaa gggcgagacc 480

cacaaggccc tgaagctgaa ggacggcggc cactacctgg tggagttcaa gtccatctac 540

atggccaaga agcccgtgca gctgcccggc tactactacg tggacgccaa gctggacatc 600

acctcccaca acgaggacta caccatcgtg gagcagtacg agcgcaccga gggccgccac 660

cacctgttcc tg 672

<210> 36
<211> 708
<212> DNA
<213> Artificial sequence

<220>
<223> Nucleic acid sequence encoding mCherry

<400> 36
atggtgagca agggcgagga ggataacatg gccatcatca aggagttcat gcgcttcaag 60

gtgcacatgg agggctccgt gaacggccac gagttcgaga tcgagggcga gggcgagggc 120

cgcccctacg agggcaccca gaccgccaag ctgaaggtga ccaagggtgg ccccctgccc 180

ttcgcctggg acatcctgtc ccctcagttc atgtacggct ccaaggccta cgtgaagcac 240

cccgccgaca tccccgacta cttgaagctg tccttccccg agggcttcaa gtgggagcgc 300

gtgatgaact tcgaggacgg cggcgtggtg accgtgaccc aggactcctc cctgcaggac 360

ggcgagttca tctacaaggt gaagctgcgc ggcaccaact tcccctccga cggccccgta 420

atgcagaaga agaccatggg ctgggaggcc tcctccgagc ggatgtaccc cgaggacggc 480

gccctgaagg gcgagatcaa gcagaggctg aagctgaagg acggcggcca ctacgacgct 540

gaggtcaaga ccacctacaa ggccaagaag cccgtgcagc tgcccggcgc ctacaacgtc 600

aacatcaagt tggacatcac ctcccacaac gaggactaca ccatcgtgga acagtacgaa 660

cgcgccgagg gccgccactc caccggcggc atggacgagc tgtacaag 708

<210> 37
<211> 23
<212> DNA
<213> Artificial sequence

<220>
<223> DNA sequence of the recognition site for sgRNA

<400> 37
cccaaaagga ggatcgtact tgg 23

<210>  38
<211>  23
<212>  PRT
<213>  Artificial sequence

<220>
<223>  Influenza virus HA2

<400>  38

Gly Leu Phe Gly Ala Ile Ala Gly Phe Ile Glu Asn Gly Trp Glu Gly
1               5                   10                  15

Met Ile Asp Gly Trp Tyr Gly
            20

<210>  39
<211>  30
<212>  PRT
<213>  Artificial sequence

<220>
<223>  KALA

<400>  39

Trp Glu Ala Lys Leu Ala Lys Ala Leu Ala Lys Ala Leu Ala Lys His
1               5                   10                  15

Leu Ala Lys Ala Leu Ala Lys Ala Leu Lys Ala Cys Glu Ala
            20                  25                  30

<210>  40
<211>  30
<212>  PRT
<213>  Artificial sequence

<220>
<223>  GALA

<400>  40

Trp Glu Ala Ala Leu Ala Glu Ala Leu Ala Glu Ala Leu Ala Glu His
1               5                   10                  15

Leu Ala Glu Ala Leu Ala Glu Ala Leu Glu Ala Leu Ala Ala
            20                  25                  30

<210>  41
<211>  26
<212>  PRT
<213>  Artificial sequence

<220>
<223>  Melittin

<400>  41

Gly Ile Gly Ala Val Leu Lys Val Leu Thr Thr Gly Leu Pro Ala Leu
1               5                   10                  15

Ile Ser Trp Ile Lys Arg Lys Arg Gln Gln
            20                  25

<210>  42
<211>  16
<212>  PRT
<213>  Artificial sequence

<220>
<223>  Penetratin

<400>  42

Arg Gln Ile Lys Ile Trp Phe Gln Asn Arg Arg Met Lys Trp Lys Lys
1               5                   10                  15

<210>  43
<211>  11
<212>  PRT
<213>  Artificial sequence

<220>
<223>  HIV-TAT(47-57)

<400>  43

Tyr Gly Arg Lys Lys Arg Arg Gln Arg Arg Arg
1               5                   10

<210>  44
<211>  8
<212>  PRT
<213>  Artificial sequence

<220>
<223>  HIV-1 Rev(34-50)

<400>  44

Lys Gln Ala Ile Pro Val Ala Lys
1               5

<210>  45
<211>  34
<212>  PRT
<213>  Artificial sequence

<220>
<223>  VP22

<400>  45

Asp Ala Ala Thr Ala Thr Arg Gly Arg Ser Ala Ala Ser Arg Pro Thr

1               5                   10                  15

Glu Arg Pro Arg Ala Pro Ala Arg Ser Ala Ser Arg Pro Arg Arg Pro
            20                  25                  30

Val Glu

<210>  46
<211>  27
<212>  PRT
<213>  Artificial sequence

<220>
<223>  Transportan

<400>  46

Gly Trp Thr Leu Asn Ser Ala Gly Tyr Leu Leu Gly Lys Ile Asn Leu
1               5                   10                  15

Lys Ala Leu Ala Ala Leu Ala Lys Lys Ile Leu
            20                  25

<210>  47
<211>  21
<212>  PRT
<213>  Artificial sequence

<220>
<223>  Pep-1

<220>
<221>  MOD_RES
<222>  (1)..(1)
<223>  ACETYLATION

<220>
<221>  MOD_RES
<222>  (21)..(21)
<223>  AMIDATION (-NH-CH2-CH2-SH)

<400>  47

Lys Glu Thr Trp Trp Glu Thr Trp Trp Thr Glu Trp Ser Gln Pro Lys
1               5                   10                  15

Lys Lys Arg Lys Val
            20

<210>  48
<211>  15
<212>  PRT
<213>  Artificial sequence

<220>
<223>  Pep-7

<400>  48

Ser Asp Leu Trp Glu Met Met Met Val Ser Leu Ala Cys Gln Tyr
1               5                   10                  15

<210>  49
<211>  29
<212>  PRT
<213>  Artificial sequence

<220>
<223>  Leucine zipper NZ

<400>  49

Ala Leu Lys Lys Glu Leu Gln Ala Asn Lys Lys Glu Leu Ala Gln Leu
1               5                   10                  15

Lys Trp Glu Leu Gln Ala Leu Lys Lys Glu Leu Ala Gln
            20                  25

<210>  50
<211>  30
<212>  PRT
<213>  Artificial sequence

<220>
<223>  Leucine zipper CZ

<400>  50

Glu Gln Leu Glu Lys Lys Leu Gln Ala Leu Glu Lys Lys Leu Ala Gln
1               5                   10                  15

Leu Glu Trp Lys Asn Gln Ala Leu Glu Lys Lys Leu Ala Gln
            20                  25                  30

<210>  51
<211>  87
<212>  DNA
<213>  Artificial sequence

<220>
<223>  Nucleic acid sequence encoding leucine zipper NZ

<400>  51
gcactgaaaa aagaactgca ggcaaataaa aaagaactgg cacagctgaa atgggaactg      60

caggcactga aaaaagaact ggcacag                                          87

<210>  52
<211>  90
<212>  DNA

<213>  Artificial sequence

<220>
<223>  Nucleic acid sequence encoding leucine zipper CZ

<400>  52
gaacagctgg aaaaaaaact gcaggcactg gaaaaaaaac tggcacagct ggaatggaaa      60

aatcaggcac tggaaaaaaa actggcacag                                        90


<210>  53
<211>  109
<212>  PRT
<213>  Artificial sequence

<220>
<223>  TrxA amino acid sequence

<400>  53

Met Ser Asp Lys Ile Ile His Leu Thr Asp Asp Ser Phe Asp Thr Asp
1               5                   10                  15


Val Leu Lys Ala Asp Gly Ala Ile Leu Val Asp Phe Trp Ala Glu Trp
            20                  25                  30


Cys Gly Pro Cys Lys Met Ile Ala Pro Ile Leu Asp Glu Ile Ala Asp
        35                  40                  45


Glu Tyr Gln Gly Lys Leu Thr Val Ala Lys Leu Asn Ile Asp Gln Asn
    50                  55                  60


Pro Gly Thr Ala Pro Lys Tyr Gly Ile Arg Gly Ile Pro Thr Leu Leu
65                  70                  75                  80


Leu Phe Lys Asn Gly Glu Val Ala Ala Thr Lys Val Gly Ala Leu Ser
                85                  90                  95


Lys Gly Gln Leu Lys Glu Phe Leu Asp Ala Asn Leu Ala
            100                 105


<210>  54
<211>  327
<212>  DNA
<213>  Artificial sequence

<220>
<223>  TrxA nucleic acid sequence

<400>  54
atgagcgata aaattattca cctgactgac gacagttttg acacggatgt actcaaagcg      60

gacggggcga tcctcgtcga tttctgggca gagtggtgcg gtccgtgcaa aatgatcgcc     120

ccgattctgg atgaaatcgc tgacgaatat cagggcaaac tgaccgttgc aaaactgaac     180

atcgatcaaa accctggcac tgcgccgaaa tatggcatcc gtggtatccc gactctgctg 240

ctgttcaaaa acggtgaagt ggcggcaacc aaagtgggtg cactgtctaa aggtcagttg 300

aaagagttcc tcgacgctaa cctggcc 327

## Description

### Technical Field

The present invention relates to the field of molecular biology, and more specifically, to a fusion protein and complex for intracellular delivery of a cargo molecule.

### Background Art

Cell membrane as a selectively permeable barrier is essential for cell survival and function. Although small molecules can pass through cell membrane through the natural process of cell or the direct diffusion of lipid bilayer, in most cases, the effective passage of intracellular cargo, for example exogenous active biomacromolecule, through the plasma membrane is still a major obstacle to cell transport process. Therefore, a molecular transport tool that can effectively improve the transport efficiency of intracellular cargo to a living cell is extremely important for its application in biomedicine and other fields.

Cell-penetrating peptides (CPPs) are currently one of the most common and effective carriers used to achieve the process of cellular uptake. Cell penetrating peptides, typically containing 5 to 30 amino acids, can carry a biomacromolecule through the cell membrane and into the cell by means of chemical cross-linking, fusion expression or non-covalent binding. So far, hundreds of CPPs derived from natural proteins or artificially synthesized have been reported and used in the intracellular delivery of intracellular cargo, and can be divided into three categories according to their chemical properties: (1) cationic CPPs, which are rich in arginine and lysine residues, and have strong positive charges at physiological pH; (2) amphipathic CPPs, which comprise polar and non-polar regions, and in addition to lysines and arginines distributed throughout their sequences, they are also rich in hydrophobic residues, such as valine, leucine, isoleucine and alanine; (3) hydrophobic CPPs, which mainly comprise non-polar amino acids. CPPs interact with negative charge on cell membrane surface or hydrophobic lipid bilayers through their strong positive charges or hydrophobic groups; when carrying a small molecule, they can directly translocate across the cell membrane in a non-energy-consuming way, and when carrying a biomacromolecule, they enter the cell basically by energy-dependent endocytosis. Due to the advantages such as low dosage, short transport time, controllable dose, simple operation, low toxicity and side effects of CPP-mediated cell entry, CPPs have currently been widely used both in vitro and in vivo to transport polypeptides, proteins, oligonucleotides, plasmids, liposomes and metal ions, small molecule fluorescence, nanoparticles, etc.

Although CPPs have many advantages in transporting biomacromolecules, there are still some limitations, in which the first problem to be solved is their low transmembrane delivery efficiency. At present, it is generally believed that when intracellular cargo is transported into a cell, CPPs interact with the surface of cell membrane and then enter the cell through endocytosis. Cell endocytosis can be divided into four main types according to endocytosis mechanism thereof: macropinocytosis, clathrin-mediated endocytosis, caveolae/lipid raft-mediated endocytosis and clathrin/caveolae-independent endocytosis. All of these different types of endocytosis will eventually generate an endosome, where the maturation from early endosome to late endosome is accompanied by a gradual decrease of pH in endosome, and finally fused with lysosome. Endosomal escape of intracellular cargo into the cytoplasm before entering the lysosome is required, otherwise, the intracellular cargo will eventually enter the lysosome and be degraded and cannot function. Studies have shown that only 1% of the biomacromolecules carried by CPPs can escape successfully from endosomes, and most of them are eventually directed to lysosomes and degraded. Therefore, the escape of intracellular cargo is a key limiting factor in the intracellular delivery process of CPPs, and its efficiency determines the overall delivery efficiency.

For improving the escape efficiency from endosomes, the main strategy is to destroy the integrity of the endosome membrane in the process of maturation and acidification, so that its content (comprising intracellular cargo which is to be delivered) is released into the cytoplasm. It has been reported that a buffer, such as chloroquine, methylamine and ammonium chloride, can be added to the system to physically promote the penetration and rupture of endosomes, but its strong cytotoxicity hinders its clinical application. At present, the most effective way is to use pH-sensitive fusogenic peptides derived from viruses, bacteria, animals, plants or humans. The pH-sensitive fusogenic peptides contain a certain proportion of hydrophobic amino acids, and their conformation changes drastically at low pH. During the process of maturation and acidification of endosomes after CPP-mediated endocytosis, the pH-sensitive fusogenic peptides coupled to CPPs undergo conformational change and bind to the lipid bilayers of endosome membrane once the pH value drops to a critical point, thereby severely disturbing the integrity of the phospholipid bilayer membrane and forming pores thereon or lysing the endosome membrane, and finally releasing the biomacromolecule to be delivered into the cytoplasm. At present, the most commonly used pH-sensitive fusogenic peptides are a peptide from the stem region of influenza virus hemagglutinin antigen (hereinafter referred to as HA2), and INF7 which is artificially modified based on HA2 having lower toxicity and better membrane-breaking effect, and both of them are capable of improving the escape efficiency of a protein molecule to be transported from endosomes after they are fused with CPPs and the protein molecule. However, although the macromolecular intracellular delivery mediated by CPPs fused with pH-sensitive fusogenic peptides indeed has an improved escape efficiency from endosomes, a considerable part of the macromolecules to be transported still remain in the endosomes (e.g., when fluorescent protein is used as the macromolecule to be transported, obvious dot-like distribution can be seen).

Therefore, there is still a need to develop a new delivery system to achieve the efficient release of a biomacromolecule to be transported from an endosome.

### Contents of the present invention

After a lot of experiments and repeated explorations, the inventors of the present application surprisingly found that a combination of a pH-sensitive peptide and a specific protease recognition sequence as a delivery carrier could significantly improve the release of a cargo molecule from an endosome, thereby significantly improving the cytoplasmic delivery efficiency of the cargo molecule, enabling the cargo molecule to give full play to their corresponding biological functions. Based on these findings, the present inventors have developed a carrier system that can achieve high-efficiency cytoplasmic delivery.

### Fusion protein

Therefore, the first aspect of the present invention provides a fusion protein, comprising a cell-penetrating peptide, a pH-sensitive fusogenic peptide and a protease recognition sequence, wherein the protease is selected from furin protease and/or lysosomal cysteine protease.

In some embodiments, the furin recognition sequence comprises or consists of the following sequence: R-X₁-X₂-R^{↓} (SEQ ID NO: 1), wherein X₁ is an arbitrary amino acid, X₂ is K or R, and ↓ indicates a cleavage site.

In certain embodiments, the furin recognition sequence comprises or consists of the following sequence: R-R-X₁-X₂-R^{↓} (SEQ ID NO: 2).

In certain embodiments, X₁ is selected from alanine (A), arginine (R), aspartic acid (D), cysteine (C), glutamine (Q), glutamic acid (E), histidine (H), isoleucine (I), glycine (G), aspartic acid (N), leucine (L), lysine (K), methionine (M), phenylalanine (F), proline (P), serine (S), threonine (T), tryptophan (W), tyrosine (Y) and valine (V).

In certain embodiments, the furin recognition sequence comprises or consists of the following sequence: RRHKR^{↓} (SEQ ID NO: 3).

In certain embodiments, the furin recognition sequence comprises or consists of the following sequence: QSVASSRRHKR^{↓}FAGV (SEQ ID NO: 4).

In certain embodiments, the lysosomal cysteine protease is selected from cathepsin B, cathepsin C, cathepsin X, cathepsin S, cathepsin L, cathepsin D, or cathepsin H.

In certain embodiments, the lysosomal cysteine protease is cathepsin L.

In certain embodiments, the cathepsin L recognition sequence comprises or consists of the following sequence: NNTHDLVGDVRLAGV (SEQ ID NO: 6).

In certain embodiments, the protease recognition sequence comprises a furin recognition sequence and a cathepsin L recognition sequence. In certain embodiments, the protease recognition sequence is a single-chain polypeptide, which comprises a furin recognition sequence and a cathepsin L recognition sequence from N-terminus to C-terminus, or which comprises a cathepsin L recognition sequence and a furin recognition sequence from N-terminus to C-terminus.

In certain embodiments, the protease recognition sequence comprises *RRHKR* (SEQ ID NO: 3) and NNTHDLVGDVRLAGV (SEQ ID NO: 6). In certain embodiments, the protease recognition sequence comprises SEQ ID NO: 4 and SEQ ID NO: 6.

In the present invention, the terms "pH-sensitive fusogenic peptide" and "pH-sensitive peptide" are used interchangeably, which refers to a kind of polypeptide that can undergo conformational change under an acidic condition (for example, pH<6.5) so as to promote the fusion with endosome membrane. During the process of maturation and acidification of endosome after a pH-sensitive peptide is endocytosed by a cell, once the pH drops to a critical point, such peptide will undergo conformational change and bind to the lipid bilayer of the endosome membrane so as to severely disturb the integrity of phospholipide bilayer membrane, form small pores or cause the lysis of endosome membrane, thereby releasing the transported biomacromolecule into the cytoplasm. Such polypeptide is well known in the art and is described in, for example, Varkouhi, Amir K., et al. Journal of Controlled Release 151.3 (2011): 220-228; Erazo-Oliveras A, Muthukrishnan N, Baker R, et al. Pharmaceuticals, 2012, 5(11): 1177-1209, and all of which are incorporated herein by reference.

The pH-sensitive peptide that can be used in the fusion protein of the present invention can be selected from the following proteins or polypeptides, or derived from the following proteins or polypeptides:
derived form viral protein sources: HA2 (influenza virus) and its mutants KALA, GALA; penton base (adenovirus or rhinovirus), gp41 (HIV), L2 (papilloma virus), envelope protein (West Nile virus);
derived from bacterial protein sources: listeriolysin O (LLO), pneumococcal pneumolysin (PLO), streptococcal streptolysin O (SLO), diphtheria toxin, Pseudomonas aeruginosa exotoxin A, Shiga toxin, cholera toxin;
derived from plant protein sources: ricin, saporin, gelonin;
derived from human/animal protein sources: human calcitonin, fibroblast growth factor receptor, melittin;
artificially synthesized peptides: (R-Ahx-R)(4) AhxB, penetratin (pAntp), EB1, bovine prion protein (bPrPp), sweet arrow peptide (SAP), poly(L-histidine), proline-rich peptide (proline-rich).

In certain embodiments, the pH-sensitive fusogenic peptide is selected from influenza virus HA2 (SEQ ID NO: 38) or a mutant thereof, melittin (SEQ ID NO: 41), and any combination thereof. In certain embodiments, the mutant of the influenza virus HA2 is selected from INF7 (SEQ ID NO: 8), KALA (SEQ ID NO: 39) or GALA (SEQ ID NO: 40).

In certain embodiments, the pH-sensitive fusogenic peptide comprises INF7. In certain embodiments, the pH-sensitive fusogenic peptide comprises or consists of the following sequence: SEQ ID NO: 8.

In the present invention, the term "cell penetrating peptide (CPP)" is also called "protein translocation domain (PTD), "Trojan horse peptide" or "transduction peptide" and the like, which refers to a polypeptide that can promote cellular uptake of various molecules (for example, various macromolecules including proteins or nucleic acids). Such polypeptides are well known in the art and are described in, for example, Stewart KM, et al. Org Biomol Chem. 2008 Jul 7;6(13):2242-55 and Chinese patent application: CN101490081A (all of which are incorporated herein by reference); or can be obtained by methods known in the art, such as the method described in US patent application: US 2008/0234183, which are fully incorporated herein by reference.

The CPP that can be used in the fusion protein of the present invention includes but is not limited to: cationic types: penetratin, HIV-TAT-47-57, HIV-1 Rev 34-50, FHV coat-35-49, oligoarginine (R9-R12), CCMV Gag-7-25, S413-PV, VP22, BP16, DPV3, DPV6, FAH coat, protamine 1, human cJun, Engrailed-2, Islet-1, HoxA-13, TP10, etc.; amphipathic types: transportan, Transportan 10, Pep-1, MPGα, MPGβ, CADY, Pepfect6, Pepfect14, Pepfect15, NickFect, Hel, sC18, pVEC, ARF (1-22), *YTA2,* PARI (*Palmitoyl-SFLLRN*)*,* F2Pal10 (*Palmitoyl-SFLLRN*)*,* BprPp (1-30), hLF peptide (19-40), Buforin 2, Crotamine, *Azurin* p18, hCT peptide (18-32), S413-PVrev, etc.; hydrophobic types: Kaposi's sarcoma fibroblast growth factor, Signal sequence of Ig light chain from *Caiiman crocodylus,* integrin β3 fragment, Grb2-SH₂ domain, HIV-1 gp41 (1-23), HBV translocation motif, sperm-egg fusion protein (89-111), human calcitonin (9-32), Pep-7, C105Y, K-FGF, etc.

In addition, the CPP used in the fusion protein of the present invention can also be selected from polypeptide sequences that have a sequence identity of about 60%, 70%, 80%, 90%, 95%, 99%, or 100% compared with any of the polypeptide sequences as described above, as long as the polypeptide sequence still retain its biological activity to promote the cellular uptake of molecules.

In certain embodiments, the cell-penetrating peptide is selected from the group consisting of penetratin (SEQ ID NO: 42), Tat-derived peptide, Rev(34-50) (SEQ ID NO: 44), VP22 (SEQ ID NO: 45), transportan (SEQ ID NO: 46), Pep-1 (SEQ ID NO: 47), Pep-7 (SEQ ID NO: 48), and any combination thereof. In certain embodiments, the Tat-derived peptide is selected from Tat (48-60) (SEQ ID NO: 10) or Tat (47-57) (SEQ ID NO: 43).

In certain embodiments, the cell-penetrating peptide comprises a Tat-derived peptide, such as Tat(48-60). In certain embodiments, the cell-penetrating peptide comprises or consists of the following sequence: SEQ ID NO: 10.

In some embodiments, the fusion protein of the present invention comprises the pH-sensitive fusogenic peptide, cell-penetrating peptide, and protease recognition sequence from N-terminus to C-terminus. In certain embodiments, the fusion protein comprises the pH-sensitive fusogenic peptide, cell-penetrating peptide, furin recognition sequence, and cathepsin L recognition sequence from N-terminus to C-terminus. In certain embodiments, the fusion protein comprises the pH-sensitive fusogenic peptide, cell penetrating peptide, cathepsin L recognition sequence, and furin recognition sequence from N-terminus to C-terminus.

In other embodiments, the fusion protein of the present invention comprises the cell-penetrating peptide, pH-sensitive fusogenic peptide and protease recognition sequence from N-terminus to C-terminus. In certain embodiments, the fusion protein comprises the cell penetrating peptide, pH-sensitive fusogenic peptide, furin recognition sequence, and cathepsin L recognition sequence from N-terminus to C-terminus. In certain embodiments, the fusion protein comprises the cell-penetrating peptide, pH-sensitive fusogenic peptide, cathepsin L recognition sequence, and furin recognition sequence from N-terminus to C-terminus.

In certain exemplary embodiments, the fusion protein comprises a sequence selected from or consists of a sequence shown in any one of SEQ ID NOs: 12-14.

In certain embodiments, the fusion protein may further comprises a protein tag at its N-terminus. In some embodiments, the protein tag has a solubilizing effect. In certain embodiments, the protein tag is selected from TrxA, SUMO, NusA, MBP, GST.

The second aspect of the present invention also provides a fusion protein, which further comprises a specific binding sequence on the basis of the fusion protein described in the first aspect, wherein the specific binding sequence allows an additional molecule (such as polypeptide, protein or nucleic acid) (e.g., DNA)) specifically bind to it. As used herein, the term "specific binding" or "specifically bind" refers to a non-random binding reaction between two molecules, such as a reaction between an antibody (or an antigen-binding fragment thereof) and an antigen (or epitope) to which it is directed, or a reaction between two amino acid sequences (for example, two antiparallel leucine-zipper domains) to form a heterodimer.

In certain embodiments, the specific binding sequence comprises a leucine zipper peptide, and the leucine zipper peptide is capable of forming a heterodimer with its complementary peptide. In certain embodiments, the specific binding sequence comprises a leucine zipper NZ or CZ. It is known in the art that the leucine zippers NZ and CZ are complementary peptides of each other, and there is a strong interaction between the two, so that the antiparallel leucine zippers NZ and CZ can form a heterodimer. In certain embodiments, the leucine zipper NZ comprises a sequence shown in SEQ ID NO: 49. In certain embodiments, the leucine zipper CZ comprises a sequence shown in SEQ ID NO:50.

In some embodiments, the specific binding sequence comprises a sequence shown in SEQ ID NO: 49. In other embodiments, the specific binding sequence comprises a sequence shown in SEQ ID NO:50.

In certain embodiments, the specific binding sequence is located at the C-terminus of the protease recognition sequence. In certain embodiments, the fusion protein comprises the specific binding sequence at its C-terminus.

In certain embodiments, the fusion protein may further comprises a protein tag at its N-terminus. In some embodiments, the protein tag has a solubilizing effect. In certain embodiments, the protein tag is selected from TrxA, SUMO, NusA, MBP, GST.

### Preparation of fusion protein

The fusion protein of the present invention can be prepared by various methods known in the art, for example, by genetic engineering methods (recombinant technology), or by chemical synthesis methods (for example, Fmoc solid phase method). The fusion protein of the present invention is not limited by its production method.

Therefore, in another aspect, the present invention provides an isolated nucleic acid molecule, which comprises a nucleotide sequence encoding the fusion protein of the first or second aspect of the present invention.

In another aspect, the present invention provides a vector (such as cloning vector or expression vector), which comprises the isolated nucleic acid molecule as described above. In certain embodiments, the vector is, for example, a plasmid, cosmid, phage, or the like.

In another aspect, the present invention provides a host cell, which comprises the isolated nucleic acid molecule or vector as described above. Such host cell includes, but is not limited to, prokaryotic cell such as *E. coli* cell, as well as eukaryotic cell such as yeast cell, insect cell, plant cell and animal cell (e.g., mammalian cell, such as mouse cell, human cell, etc.).

In another aspect, there is provided a method for preparing the fusion protein of the first or second aspect of the present invention, which comprises: culturing the host cell as described above under a condition that allows the expression of the fusion protein, and recovering the fusion protein from a culture of the cultured host cell.

### Complex

In another aspect, the present invention provides a complex, which comprises the fusion protein of the first or second aspect of the present invention and a cargo molecule (cargo). The cargo molecule can be any molecule.

In certain embodiments, the cargo molecule is selected from the group consisting of nucleic acid, peptide or protein, carbohydrate, lipid, chemical compound, and any mixture thereof.

In certain embodiments, the nucleic acid is selected from the group consisting of DNA molecule, RNA molecule, siRNA, antisense oligonucleotide, ribozyme, aptamer, and any combination thereof.

In certain embodiments, the cargo molecule has a molecular weight of less than 10000 Da, for example, less than 5000 Da, less than 3000 Da, or less than 1000 Da.

In some embodiments, the cargo molecule comprises a detectable label, such as enzyme, radionuclide, fluorescent dye, chemiluminescent substance, or biotin, and the like.

In certain embodiments, the cargo molecule comprises an epitope tag, reporter gene sequence, and/or nuclear localization signal (NLS) sequence. In certain embodiments, the cargo molecule is a peptide or protein.

The epitope tag that can be used in the cargo molecule is well known to those skilled in the art, examples of which include but are not limited to: His, V5, FLAG, HA, Myc, VSV-G, Trx, etc., and those skilled in the art know how to select an appropriate epitope tag according to the desired purpose (for example, purification, detection or tracing). In certain exemplary embodiments, the cargo molecule comprises a His tag.

The reporter gene sequence that can be used in the cargo molecule is well known to those skilled in the art, and examples of which include but are not limited to GST, HRP, CAT, GFP, HcRed, DsRed, CFP, YFP, BFP, etc.

The nuclear localization signal (NLS) sequence that can be used in the cargo molecule is well known to those skilled in the art, and examples thereof include but are not limited to: the NLS of SV40 virus large T antigen. In certain exemplary embodiments, the NLS sequence is shown in SEQ ID NO: 15.

In some embodiments, the fusion protein of the present invention is fused to the cargo molecule, wherein the cargo molecule is a peptide or protein. In certain embodiments, the fusion protein is as defined in the first aspect.

In certain embodiments, the cargo molecule is fused to the N-terminus or C-terminus of the fusion protein. In certain embodiments, the cargo molecule is fused to the C-terminus of the fusion protein.

In some embodiments, the complex of the present invention comprises a single-chain polypeptide, which comprises from N-terminus to C-terminus: the pH-sensitive fusogenic peptide, cell-penetrating peptide, protease recognition sequence, and cargo molecule. In certain embodiments, the single-chain polypeptide comprises the pH-sensitive fusogenic peptide, cell-penetrating peptide, furin recognition sequence, cathepsin L recognition sequence, and cargo molecule from N-terminus to C-terminus. In certain embodiments, the single-chain polypeptide comprises the pH-sensitive fusogenic peptide, cell-penetrating peptide, cathepsin L recognition sequence, furin recognition sequence, and cargo molecule from N-terminus to C-terminus.

In other embodiments, the complex of the present invention comprises a single-chain polypeptide, which comprises from N-terminus to C-terminus: the cell penetrating peptide, pH-sensitive fusogenic peptide, protease recognition sequence and cargo molecule. In certain embodiments, the single-chain polypeptide comprises the cell-penetrating peptide, pH-sensitive fusogenic peptide, furin recognition sequence, cathepsin L recognition sequence, and cargo molecule from N-terminus to C-terminus. In certain embodiments, the single-chain polypeptide comprises the cell-penetrating peptide, pH-sensitive fusogenic peptide, cathepsin L recognition sequence, furin recognition sequence, and cargo molecule from N-terminus to C-terminus.

In certain exemplary embodiments, the cargo molecule is a zinc finger protein (e.g., ZFP9), protein phosphatase (e.g., Ppm1b), or Cas effector protein (e.g., Cas9). The expression "Cas effector protein" refers to the effector protein of CRISPR-Cas system. In certain exemplary embodiments, the zinc finger protein or Cas effector protein comprises an NLS sequence.

In other embodiments, the fusion protein of the present invention is chemically coupled to the cargo molecule. The term "chemically coupled" refers to a bond obtained in a chemical reaction between a reactive group contained in the fusion protein and a reactive group contained in the cargo molecule, after which the two moieties are connected by a covalent bond. Before the above-mentioned chemical reaction (coupling reaction), the fusion protein, the cargo molecule, or both can be modified with a linker molecule in a separate reaction so that they can contain the reactive groups required for the chemical coupling respectively. The choice of linker molecule used to modify the fusion protein or cargo molecule depends on the coupling strategy used. In certain embodiments, the fusion protein is as defined in the first aspect.

In certain embodiments, the covalent bond is a disulfide bond, phosphodiester bond, phosphorothioate bond, amide bond, amine bond, thioether bond, ether bond, ester bond, or carbon-carbon bond.

In certain embodiments, the cargo molecule is coupled to the N-terminus or C-terminus of the fusion protein. In certain embodiments, the cargo molecule is coupled to the C-terminus of the fusion protein.

In certain embodiments, the cargo molecule is a nucleic acid.

In other embodiments, the fusion protein of the present invention is non-covalently linked to the cargo molecule.

In certain embodiments, the fusion protein is conjugated to the cargo molecule through an electrostatic interaction. In certain embodiments, the cargo molecule is a nucleic acid.

In some embodiments, the fusion protein is as defined in the second aspect, and the fusion protein is non-covalently linked to the cargo molecule through the specific binding sequence it contains. In such embodiments, the cargo molecule comprises a domain capable of specifically binding to the specific binding sequence in the fusion protein.

In some embodiments, the domain capable of specifically binding to the specific binding sequence in the fusion protein is an amino acid sequence.

In certain embodiments, the cargo molecule is a peptide or protein. In some embodiments, the cargo molecule comprises at its N-terminus an amino acid sequence capable of specifically binding to the specific binding sequence in the fusion protein.

In certain embodiments, the specific binding sequence in the fusion protein comprises a leucine zipper peptide, and the cargo molecule comprises the complementary peptide of the leucine zipper, so that the leucine zipper peptide and the complementary peptide can form a heterodimer.

In certain embodiments, the specific binding sequence in the fusion protein comprises a leucine zipper NZ (for example, as shown in SEQ ID NO: 49), and the cargo molecule comprises a leucine zipper CZ (for example, as shown in SEQ ID NO: 50).

In certain embodiments, the specific binding sequence in the fusion protein comprises a leucine zipper CZ (for example, as shown in SEQ ID NO: 50), and the cargo molecule comprises a leucine zipper NZ (for example, as shown in SEQ ID NO: 49).

### Preparation of complex

The complex of the present invention can be prepared by various methods known in the art, for example, by genetic engineering methods (recombinant technology), or by chemical synthesis methods (for example, Fmoc solid phase method). The complex of the present invention is not limited by the way it is produced.

In some embodiments, when the complex comprises the fusion protein and the cargo molecule which are fused together, the complex of the present invention can be obtained by genetic engineering recombination technology. For example, a DNA molecule encoding the complex can be obtained by chemical synthesis or PCR amplification. The resulting DNA molecule can be inserted into an expression vector and then transfected into a host cell. Then, the transfected host cell can be cultured under a specific condition, and the complex of the present invention can be thus expressed.

Therefore, in another aspect, the present invention provides an isolated nucleic acid molecule, which comprises a nucleotide sequence encoding the complex as described above.

In another aspect, the present invention provides a vector (e.g, cloning vector or expression vector), which comprises the isolated nucleic acid molecule as described above. In certain embodiments, the vector is, for example, a plasmid, cosmid, phage, and the like.

In another aspect, the present invention provides a host cell, which comprises the isolated nucleic acid molecule or vector as described above. Such host cell includes but is not limited to, prokaryotic cell such as *E. coli* cell, as well as eukaryotic cell such as yeast cell, insect cell, plant cell and animal cell (e.g., mammalian cell, such as mouse cell, human cell, etc.).

In another aspect, there is provided a method for preparing the complex as described above, which comprises: culturing the host cell as described above under a condition that allows the expression of the complex, and recovering the complex from a culture of the cultured host cell.

In other embodiments, when the complex comprises the fusion protein and the cargo molecule which are chemically coupled, the complex of the present invention can be obtained by the following exemplary method: mixing the fusion protein with the cargo molecule under a condition that allows the reactive groups contained in the fusion protein and the cargo molecule to undergo a chemical reaction, so that the two moieties are linked by a covalent bond. In some embodiments, the method further comprises: modifying the fusion protein, cargo molecule, or both with a linker molecule so that they each contain the reactive groups required for the above-mentioned chemical reaction. In certain embodiments, the cargo molecule is a nucleic acid.

In other embodiments, when the complex comprises the fusion protein and the cargo molecule which are conjugated by an electrostatic interaction, the complex of the present invention can be obtained by the following exemplary method: (1) mixing the fusion protein of the present invention with the cargo molecule to form a mixture; and (2) incubating the mixture so that the fusion protein and the cargo molecule form the complex. In certain embodiments, the cargo molecule is a nucleic acid.

In other embodiments, when the complex comprises the fusion protein and the cargo molecule that are linked by means of specific binding rather than covalent binding, the complex of the present invention can be obtained by the following exemplary method: (1) mixing the fusion protein of the second aspect of the present invention with the cargo molecule, in which the cargo molecule comprises a domain that specifically binds to the specific binding sequence in the fusion protein; and (2) incubating the mixture so that the fusion protein and the cargo molecule form the complex. In certain embodiments, the cargo molecule is a polypeptide or protein.

### Composition

When the fusion protein of the present invention can be linked to the cargo molecule through a non-covalent interaction, the delivery complex can be obtained by mixing the fusion protein and the cargo molecule. Therefore, in another aspect, the present invention also provides a composition comprising the fusion protein of the present invention and a cargo molecule.

In certain embodiments, the cargo molecule is selected from the group consisting of nucleic acid, peptide or protein, carbohydrate, lipid, chemical compound, and any mixture thereof. In certain embodiments, the nucleic acid is selected from the group consisting of DNA molecule, RNA molecule, siRNA, antisense oligonucleotide, ribozyme, aptamer, and any combination thereof.

In certain embodiments, the cargo molecule is selected from nucleic acid.

In certain embodiments, the cargo molecule is a polypeptide or protein.

In certain embodiments, the fusion protein is as defined in the second aspect. The cargo molecule comprises a domain capable of specifically binding to the specific binding sequence in the fusion protein.

### Application and method

The fusion protein of the present invention can efficiently release a cargo molecule from an endosome, and once the cargo molecule is available in the cytoplasm, it can play any role related thereto. Therefore, the fusion protein of the present invention can be used as an intracellular delivery agent, which can be further used in research as well as therapeutic and diagnostic applications.

Therefore, in another aspect, the present invention provides a pharmaceutical composition, which comprises the fusion protein, complex, composition, isolated nucleic acid molecule, vector or host cell of the present invention, and a pharmaceutically acceptable carrier and/or excipient.

In certain embodiments, the cargo molecule contained in the complex or composition is a pharmaceutically active agent.

In certain embodiments, the cargo molecule contained in the complex or composition is a detectable label. The label can be used for diagnosis, for research of drug disposition (for example, absorption, distribution, metabolism, excretion), for research of treatment or drug efficacy or side effects, and the like.

In another aspect, the present invention also relates to a use of the fusion protein of the present invention, or an isolated nucleic acid molecule, vector or host cell comprising a nucleotide sequence encoding the fusion protein, as a delivery agent (for example, intracellular delivery agent and/or transfection agent) in the manufacture of a medicament.

In another aspect, the present invention also relates to a use of the complex or composition of the present invention, or an isolated nucleic acid molecule, vector or host cell comprising a nucleotide sequence encoding the complex or composition, in the manufacture of a medicament for treating a disease; wherein the cargo molecule contained in the complex or composition can treat the disease.

In certain embodiments, the disease is a disease associated with programmed cell death, wherein the cargo molecule comprises protein phosphatase 1B. In certain embodiments, the disease associated with programmed cell death comprises liver injury (e.g., drug-induced liver injury), inflammatory disease, ischemia-reperfusion injury, and/or neurodegenerative disease.

The fusion protein, complex or composition, or pharmaceutical composition of the present invention can be in any form known in the medical field, for example, it can be a tablet, pill, suspension, emulsion, solution, gel, capsule, powder, granule, elixir, lozenge, suppository, injection (including injection solution, freeze-dried powder), inhalant, spray and other form. The preferred dosage form depends on the intended mode of administration and therapeutic use.

The fusion protein, complex or composition, or pharmaceutical composition of the present invention can be administered by any suitable method known in the art, including but not limited to oral, rectal, parenteral or topical administration.

An exemplary route of administration is oral administration. Liquid dosage forms for oral administration include pharmaceutically acceptable emulsion, microemulsion, solution, suspension, syrup, elixir and the like. In addition to the active ingredient, the liquid dosage form may comprise an inert diluent commonly used in the art, such as water or other solvent, solubilizer and emulsifier, such as ethanol, isopropanol, ethyl acetate, ethyl acetate, benzyl alcohol, benzyl benzoate, propylene glycol, 1,3-butanediol, dimethylformamide, oil (e.g., cottonseed oil, peanut oil, corn oil, germ oil, olive oil, castor oil and sesame oil), glycerol, tetrahydrofurfuryl alcohol, polyethylene glycol and fatty acid ester of sorbitan, and any mixture thereof. In addition to the inert diluent, the liquid dosage form for oral administration may also comprise an adjuvant, such as wetting agent, emulsifying and suspending agent, sweetening agent, flavoring agent, and perfuming agent. Solid dosage forms for oral administration include capsule, tablet, pill, lozenge, powder, granule and the like. In addition to the active ingredient, the solid dosage form may comprise a pharmaceutically acceptable inert excipient or carrier, such as filler (e.g., lactose, sucrose, glucose, mannitol, starch, microcrystalline cellulose, galactose, crospovidone and calcium sulfate); binder (e.g., carboxymethyl cellulose, alginate, gelatin, polyvinylpyrrolidone, sucrose and acacia); wetting agent (e.g., cetyl alcohol and glyceryl monostearate); disintegrant (e.g, agar, calcium carbonate, starch, alginic acid, sodium carboxymethyl cellulose, sodium carboxymethyl starch); lubricant (e.g., talc, calcium stearate, magnesium stearate, solid polyethylene glycol, sodium laurelsulphate); and any mixture thereof.

The fusion protein, complex or composition, or pharmaceutical composition of the present invention can also be administered by a non-oral route.

Therefore, another exemplary route of administration is parenteral administration, for example, subcutaneous injection, intravenous injection, intraperitoneal injection, intramuscular injection, intrastemal injection, and infusion. The dosage form for parenteral administration may be an injection preparation, including injection solution, sterile powder for injection, or concentrated solution for injection. In addition to the active ingredient, the injection dosage form may comprise a pharmaceutically acceptable carrier such as sterile water, Ringer's solution and isotonic sodium chloride solution, and an appropriate additive such as antioxidant, buffer and bacteriostatic agent may also be added according to the properties of drug.

Another exemplary route of administration is topical administration, such as transdermal administration (e.g., administration via transdermal patch or iontophoresis device), intraocular administration, or intranasal or inhalation administration. The dosage form for transdermal administration can be a topical gel, spray, ointment and cream. In addition to the active ingredient, the topical dosage form may comprise an ingredient that enhances the absorption or penetration of the active compound through skin or other action area.

Another exemplary route of administration is rectal administration. The dosage form for rectal administration may be a suppository.

In addition, other carrier materials and administration methods known in the pharmaceutical field can also be used. The fusion protein, complex or composition, or pharmaceutical composition of the present invention can be prepared by any known pharmaceutical process, such as effective methods for formulation and administration.

In another aspect, the present invention provides a kit, which comprises the fusion protein, complex, composition, isolated nucleic acid molecule, vector or host cell of the present invention. In certain embodiments, the kit further comprises an instruction for transfection and/or intracellular delivery. In certain embodiments, the kit is used for transfection and/or intracellular delivery of a cargo molecule (e.g., nucleic acid, peptide or protein, carbohydrate, lipid, chemical compound, and any mixture thereof). In certain embodiments, the cell is a mammalian cell, such as a human cell.

In another aspect, the present invention also relates to a use of the fusion protein, complex, composition, isolated nucleic acid molecule, vector or host cell of the present invention as a delivery reagent (for example, transfection reagent or intracellular delivery reagent). In certain embodiments, the delivery agent is used for intracellular delivery of a cargo molecule (e.g., nucleic acid, peptide or protein, carbohydrate, lipid, chemical compound, and any mixture thereof). In certain embodiments, the cell is a mammalian cell, such as a human cell.

In another aspect, the present invention provides a method for delivering a molecule into a cell, which comprises contacting the cell with the complex of the present invention, wherein the complex comprises the molecule.

In certain embodiments, the contacting of the cell with the complex is performed in vivo.

In certain embodiments, the contacting of the cell with the complex is performed ex vivo.

In certain embodiments, the contacting of the cell with the complex is performed in vitro.

In certain embodiments, the cell is an eukaryotic cell, such as a mammalian cell, such as a human cell.

### Definition of Terms

In the present invention, unless otherwise specified, the scientific and technical terms used herein have the meanings commonly understood by those skilled in the art. Moreover, the laboratory procedures for the cell culture, biochemistry, nucleic acid chemistry, immunology used herein are all routine steps widely used in the corresponding fields. At the same time, in order to better understand the present invention, definitions and explanations of related terms are provided below.

As used herein, the term "isolated" refers to being obtained from a natural state by artificial means. If a certain "isolated" substance or component appears in nature, it may be that the natural environment in which it is located has changed or the substance has been separated from the natural environment, or the both occur. For example, for a certain un-isolated polynucleotide or polypeptide that is naturally present in a living animal, the same polynucleotide or polypeptide with high purity isolated from this natural state is called "isolated". The term "isolated" does not exclude the presence of an artificial or synthetic substance, nor does it exclude the presence of other impure substances that do not affect the activity of the substance.

As used herein, the term "vector" refers to a nucleic acid delivery vehicle into which a polynucleotide can be inserted. When the vector can express a protein encoded by an inserted polynucleotide, the vector is called an expression vector. The vector can be introduced into a host cell through transformation, transduction or transfection, so that a genetic material element it carries can be expressed in the host cell. Vectors are well-known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); phage such as lambda phage or M13 phage, and animal virus. Animal viruses that can be used as vectors include, but are not limited to, retrovirus (including lentivirus), adenovirus, adeno-associated virus, herpes virus (e.g., herpes simplex virus), poxvirus, baculovirus, papillomavirus, and papovavirus (such as SV40). A vector can contain a variety of elements that control expression, including but not limited to promoter sequence, transcription initiation sequence, enhancer sequence, selection element, and reporter gene. In addition, the vector may also contain a replication origin.

As used herein, the term "host cell" refers to a cell into which a vector can be introduced, including but not limited to, prokaryotic cell such as *Escherichia coli* or *Bacillus subtilis,* fungal cell such as yeast cell or *Aspergillus,* insect cell such as S2 *Drosophila* cell or Sf9, or animal cell such as fibroblast, CHO cell, COS cell, NSO cell, HeLa cell, BHK cell, HEK 293 cell or human cell.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The twenty conventional amino acids involved herein are expressed in routine manners. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and can be used interchangeably. And in the present invention, amino acids are usually represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which are well-known in the art (see, for example, Remington's Pharmaceutical Sciences. Edited by Gennaro AR, 19th ed. Pennsylvania: Mack Publishing Company, 1995), and include, but are not limited to: pH adjuster, surfactant, ionic strength enhancer, agent for maintaining osmotic pressure, agent for delaying absorption, diluent, adjuvant, preservative, stabilizer, etc. For example, pH adjusting agent includes but is not limited to phosphate buffer. Surfactant includes but is not limited to cationic, anionic or non-ionic surfactant, such as Tween-80. Ionic strength enhancer includes but is not limited to sodium chloride. Agent for maintaining osmotic pressure includes but is not limited to, sugar, NaCl and the like. Agent for delaying absorption includes but is not limited to monostearate and gelatin. Diluent includes but is not limited to water, aqueous buffer (e.g., buffered saline), alcohol and polyol (e.g., glycerol), etc. Adjuvant includes but is not limited to aluminum adjuvant (e.g., aluminum hydroxide), Freund's adjuvant (e.g, complete Freund's adjuvant), and the like. Preservative includes but is not limited to various antibacterial and antifungal agents, such as thimerosal, 2-phenoxyethanol, paraben, trichloro-tert-butanol, phenol, sorbic acid and the like. Stabilizer has the meaning commonly understood by those skilled in the art, which can stabilize a desired activity of an active ingredient in a drug (for example, the inhibitory activity of PSD-95 ubiquitination), including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dried whey, albumin or casein) or degradation product thereof (e.g., lactalbumin hydrolysate), etc.

As used herein, the term "treatment" refers to a method performed in order to obtain a beneficial or desired clinical result. For the purpose of the present invention, the beneficial or desired clinical result includes, but is not limited to, alleviating symptom, narrowing the scope of disease, stabilizing (i.e. not aggravating) the state of disease, delaying or slowing the progress of disease, and alleviating symptoms (either partially or completely), no matter detectable or not detectable. In addition, "treatment" can also refer to prolonging survival time as compared to an expected survival time (if not receiving treatment).

As used herein, the term "subject" refers to a mammal, such as a primate mammal, such as a human.

### Beneficial effects of the present invention

The delivery system of the present invention can significantly improve the release of cargo molecule from endosome, thereby significantly improving the cytoplasmic delivery efficiency of the cargo molecule, so that the cargo molecule can fully exert its corresponding biological function. Therefore, the delivery system of the present invention provides an effective means for influencing the biological mechanism and pathway of cell, can be used in many fields such as research, treatment, diagnosis, and has broad application prospects and clinical value.

The embodiments of the present invention will be described in detail below in conjunction with the accompanying drawings and examples. However, those skilled in the art will understand that the following drawings and examples are only used to illustrate the present invention, but not to limit the scope of the present invention. According to the accompanying drawings and the following detailed description of the preferred embodiments, various objects and advantageous aspects of the present invention will become apparent to those skilled in the art.

### Brief Description of the Drawings

FIG. 1 shows a schematic diagram of the principle of the Split-GFP system for detecting the escape efficiency from endosome in Example 1.
FIG. 2 shows a schematic diagram of the structure of the delivery system-GFPβ1-10 protein complex in Example 1.
FIGS. 3 to 4 show the SDS-PAGE results of the delivery system-GFPβ1-10 complex in Example 1.
FIG. 5 shows the results of flow cytometry analysis of the delivery system for transducing GFPβ1-10 in Example 1.
FIG. 6 shows the results of fluorescence microscopy observation of the delivery system for transducing GFPβ1-10 in Example 1.
FIG. 7 shows the results of flow cytometry analysis of the delivery system containing mutation in enzymatic cleavage site for transducing GFPβ1-10 in Example 1.
FIG. 8 shows the Western blot detection results of the relative proportion of the cleaved and un-cleaved/complete GFPβ1-10 in the cell and the retention time thereof in the cell after transduction by the delivery system in Example 1.
FIG. 9 shows the Western blot detection results of the relative proportion of the cleaved and un-cleaved/complete GFPβ1-10 in the cell after transduction by the delivery system containing mutation in enzymatic cleavage site in Example 1.
FIG. 10 shows the results of flow cytometry analysis of the transduction by the delivery system containing mutation in enzymatic cleavage site but no pH-sensitive peptide in Example 1.
FIG. 11 shows the Western blot detection results of the relative proportion of the cleaved and un-cleaved/complete GFPβ1-10 in the cell after transduction by the delivery system containing mutation in enzymatic cleavage site but no pH-sensitive peptide in Example 1.
FIG. 12 shows a schematic diagram of the structure of the delivery system-ZFP9 complex in Example 2.
FIG. 13 shows the results of SDS-PAGE of the delivery system-ZFP9 complex in Example 2.
FIG. 14 shows the map of the eukaryotic expression plasmid containing ZFP9 binding site in Example 2.
FIGS. 15 to 16 show the results of flow cytometry analysis of the transduction of ZFP9 by the delivery system in Example 2.
FIG. 17 shows a schematic diagram of the structure of the delivery system-Ppmlb complex in Example 3.
FIG. 18 shows the results of SDS-PAGE of the delivery system-Ppmlb complex in Example 3.
FIG. 19 shows the results of flow cytometry analysis of the effect of the delivery system-Ppm1b complex on the ratio of TNF-α-induced cell necrosis in Example 3.
FIG. 20 shows a schematic diagram of the structure of the delivery system-Cas9 complex in Example 4.
FIG. 21 shows the results of SDS-PAGE of the delivery system-Cas9 complex in Example 4.
FIG. 22 shows a schematic diagram of the principle of detection of the editing efficiency of CRISPR/Cas9 by using HEK293T-RFP reporter cells in Example 4.
FIG. 23 shows the map of RFP reporter lentiviral plasmid in Example 4.
FIG. 24 shows the results of flow cytometry analysis of the gene editing efficiency of the delivery system-Cas9 complex in Example 4.
FIG. 25 shows the principle of the delivery system based on the adapter method in Example 5.
FIG. 26 shows the clone design of recombinant protein for the delivery system based on the adapter method in Example 5.
FIG. 27 shows the purification results of recombinant protein for the delivery system based on the adapter method in Example 5.
FIG. 28 shows the evaluation results of the delivery effect of the adapter-based delivery system by the Split-GFP endosome escape system in Example 5.

### Sequence Information

The information of some sequences involved in the present invention is provided in Table 1 below.

**Table 1: Description of sequences**

| SEQ ID NO | Description |
|---|---|
| 1 | Furin recognition sequence-1 |
| 2 | Furin recognition sequence-2 |
| 3 | Furin recognition sequence-3 |
| 4 | Furin recognition sequence-4 (Ne) |
| 5 | Nucleic acid sequence encoding Ne |
| 6 | CTSL recognition sequence N |
| 7 | Nucleic acid sequence encoding N |
| 8 | INF7 |
| 9 | Nucleic acid sequence encoding INF7 |
| 10 | Tat(48-60) |
| 11 | Nucleic acid sequence encoding Tat (48-60) |
| 12 | Fusion protein TIN |
| 13 | Fusion protein TINe |
| 14 | Fusion protein TINNe |
| 15 | NLS |
| 16 | CTSL recognition sequence Na |
| 17 | CTSL recognition sequence Nb |
| 18 | Furin recognition sequence Nc |
| 19 | Furin recognition sequence Nd |
| 20 | CTSD identification sequence Nf |
| 21 | Mutant N |
| 22 | Mutant Ne |
| 23 | GFPβ1-10-NLS |
| 24 | Nucleic acid sequence encoding GFPβ1-10-NLS |
| 25 | Nucleic acid sequence encoding Histone-H3 |
| 26 | Nucleic acid sequence encoding GFPβ11 |
| 27 | ZFP9-NLS |
| 28 | Nucleic acid sequence encoding ZFP9-NLS |
| 29 | Sequence encoding BFP |
| 30 | ZFP9 binding site sequence |
| 31 | Ppm1b |
| 32 | Nucleic acid sequence encoding Ppm1b |
| 33 | Cas9-NLS |
| 34 | Nucleic acid sequence encoding Cas9-NLS |
| 35 | Nucleic acid sequence encoding dsRed |
| 36 | Nucleic acid sequence encoding mCherry |
| 37 | DNA sequence of the recognition site for sgRNA |
| 38 | Influenza virus HA2 |
| 39 | KALA |
| 40 | GALA |
| 41 | Melittin |
| 42 | Penetratin |
| 43 | HIV-TAT(47-57) |
| 44 | HIV-1 Rev(34-50) |
| 45 | VP22 |
| 46 | Transportan |
| 47 | Pep-1 |
| 48 | Pep-7 |
| 49 | Leucine zipper NZ |
| 50 | Leucine zipper CZ |
| 51 | Nucleic acid sequence encoding leucine zipper NZ |
| 52 | Nucleic acid sequence encoding leucine zipper CZ |
| 53 | TrxA amino acid sequence |
| 54 | TrxA nucleic acid sequence |

### EXEMPLES

The present invention will now be described with reference to the following examples which are intended to illustrate the present invention rather than limit the present invention.

Unless otherwise specified, the molecular biology experimental methods and immunoassay methods used in the present invention were performed basically by referring to the methods as described in J. Sambrook et al., Molecular Cloning: Laboratory Manual, 2nd Edition, Cold Spring Harbor Laboratory Press, 1989, and FM Ausubel et al., Compiled Molecular Biology Experiment Guide, 3rd edition, John Wiley & Sons, Inc., 1995; the restriction enzymes were used in accordance with the conditions recommended by the product manufacturer. Those skilled in the art know that the examples describe the present invention by way of example, and are not intended to limit the scope sought to be protected by the present invention.

The sources of the main reagents involved in the following examples are as follows:
the materials required for cloning and construction were as follows: DNA polymerase (TaKaRa, R040A), DNA recovery kit (TianGen, DP214-03), plasmid mini-kit (TianGen, DP103-03), plasmid large-scale kit (QIAGEN, 12663), 5 tubes of Gibson assembly premix (NEB, E2611L), DNA marker (ThmeroFisher, SM0331), agarose (Biowest, BW-R0100),
the materials required for large-scale protein expression were as follows: peptone (BiSIGMA-ALDRICH, T7293-1KG), yeast powder (OXOID, LP0021B), sodium chloride (Xilong Chemical, 10011012AR), IPTG (Inalco, 1758-1400),
the media required for protein purification were as follows: SP SEPHAROSE FAST FLOW (GE Healthcare, 17-0729-01), NI SEPHAROSE (GE Healthcare, 17-5268-02),
the reagents required for protein purification and storage were as follows: glycerol/glycerol/C₃H₈O₃ (SIGMA-ALDRICH, G5516), KCl (Xilong Chemical Industry, 1002007), Na₂HPO₄·12H₂O (Xilong Chemical Industry, 1001067AR), KH₂PO₄ (Xilong Chemical Industry, 1002048AR500), imidazole (SIGMA-ALDRICH, V900153), Tris base (Seebio, 183995), glucose (Xilong Chemical Industry, 1064008AR500), BCA Protein Assay Kit (Thermo Scientific, 23227);
reagents required for cell culture: FBS (GIBCO, 10099-133), DMEM (GIBCO, 11965092), trypsin (AMRESCO, 0458);
reagents required for lentivirus packaging and infection: lentivirus packaging plasmid: pCMV-VSV-G (Addgene, 8454), pRSV-Rev (Addgene, 12253), pMDLg/pRRE (Addgene, 12251); X-tremeGENE transfection reagent (Roche, 06366244001), Puromycin (InvivoGen, antpr-5), Blasticidin (InvivoGen, ant-bl-5b), polybrene (Santa Cruz, sc-134220);
plasmids related to GFPβ1-10, ZFP9, Ppm1b, dsRed, mCherry, and Histone-H3 as used in the experiments were all synthesized by Biotech. The plasmid pCasKP-hph for amplifying the Cas9 sequence (Addgene, 117232);
other reagents: TNF-α (Novoprotein, CF09), PI (ThmeroFisher, P3566),
cell lines: HEK-293T (human renal epithelial cells), L929 (mouse fibroblasts), which were purchased from ATCC.

### Example 1: Split-GFP system-based evaluation of endosome escape efficiency of delivery system

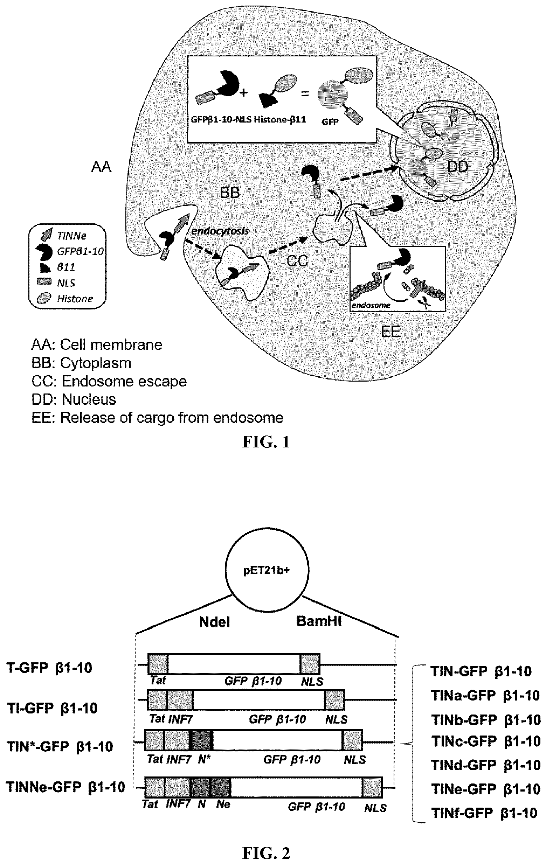

In the Split-GFP system, the eleven β-pleated sheets of GFP are split into a large fragment (β1-10) and a small fragment (β11), both of which lose the fluorescent activity; but if they meet, they can associate spontaneously and restore the fluorescence performance of GFP. Based on this, we constructed HEK293T cells stably expressing Histone-β11, and GFPβ1-10 with nuclear localization signal (NLS) as a cargo and an intracellular delivery system to be evaluated were expressed as a fusion protein, and subjected to transduction of the stable cell line. When GFPβ1-10 was transduced by the delivery system, it could bind to GFPβ11 and generate complete GFP only after successfully escaping from the endosome and entering the cytoplasm or nucleus, so that the endosome escape efficiency could be evaluated by the proportion and relative fluorescence intensity of ratio of GFP (FIG. 1).

### 1.1 Construction of delivery system-GFPβ1-10 protein complex expression vector

Construction of a recombinant protein expression vector of cargo molecule GFPβ1-10 (SEQ ID NO: 23) containing TAT (SEQ ID NO: 10), INF7 (SEQ ID NO: 8), protease cleavage site (Table 2) and nuclear localization signal (NLS) was performed, the schematic diagram of the structure of each recombinant protein was shown in FIG. 2, and the components from C-terminus to N-terminus and the amino acid sequences thereof were shown in Table 3 below. The construction method was as follows: first, the nucleic acid sequences of TAT, INF7, N, Na, Nb, Nc, Nd, Ne, Nf, mutant N, mutant Ne, and cargo molecule GFPβ1-10 in the delivery system were obtained by PCR amplification; these parts were linked through multiple rounds of PCR, and in the last round of PCR, the *NdeI* restriction site and the overlap on upstream of the corresponding *NdeI* restriction site in pET21b(+) were introduced at the 5' end of the fragment through the forward primer, and the *BamHI* restriction site and the overlap on downstream of the corresponding *BamHI* restriction site in pET-21b(+) were introduced at the 3' end of the fragment through the reverse primer. The pET-21b(+) plasmid was subjected to double digestion with *NdeI* and *BamHI.* The insert fragments with overlaps were ligated to the digested vector pET-21b(+) by GIBSON assembly.

**Table 2: Protease recognition sequences**

| Protease | Recognition sequence | SEQ ID NO: |
|---|---|---|
| CTSL | N | 6 |
| | Mutant N | 21 |
| | Na | 16 |
| | Nb | 17 |
| Furin | Nc | 18 |
| | Nd | 19 |
| | Ne | 4 |
| | Mutant Ne | 22 |
| CTSD | Nf | 20 |

**Table 3: Components contained in delivery system-cargo molecular complex**

| Complex name | components from N-terminal to C-terminal and their sequences | | | |
|---|---|---|---|---|
| | CPP | pH-sensitive peptide | Protease recognition sequence | Cargo |
| T-GFP β1-10 | Tat SEQ ID NO:10 | None | None | GFP β1-10-NLS SEQ ID NO: 23 |
| TI-GFP β1-10 | | INF7 SEQ ID NO: 8 | | |
| TIN-GFP β1-10 | | | N | |
| TINa-GFP β1-10 | | | Na | |
| TINb-GFP β1-10 | | | Nb | |
| TINc-GFP β1-10 | | | Nc | |
| TINd-GFP β1-10 | | | Nd | |
| TINe-GFP β1-10 | | | Ne | |
| TINf-GFP β1-10 | | | Nf | |
| TINNe-GFP β1-10 | | | N+Ne | |
| TNNE-GFP β1-10 | | None | | |

### 1.2 Expression and purification of delivery system-GFPβ1-10 complex:

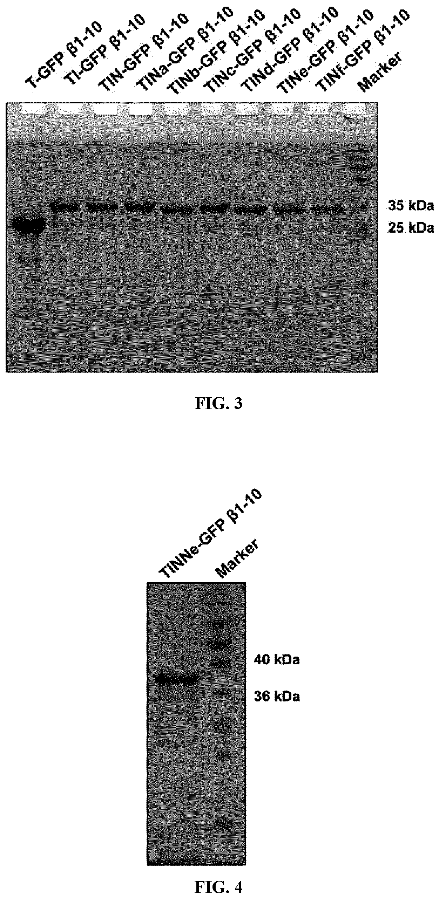

The expression plasmid described in 1.1 was transformed into the expression strain *E. coli* BL21 (DE3). A single colony was picked from the plate after transformation and inoculated in 5 ml of LB liquid medium containing ampicillin resistance and cultivated overnight, and then 1 ml of the overnight-cultured bacterial culture was transferred to 500 ml of LB liquid medium containing ampicillin resistance, followed by cultivation at 37°C and 180 rpm until the bacterial culture had OD₆₀₀ of about 0.6, and then the inducer IPTG was added to reach a final concentration of 0.2 mM, followed by induction at 25°C for 8 h. After the induction, the bacterial cells were collected after centrifugation at 7000 g for 10 min at 4°C. Then the bacterial cells were re-suspended with 10 ml of equilibration buffer for protein purification (50 ml of glycerol, 8 g of NaCl, 0.201 g of KCl, 1.44 g of Na₂HPO₄, 0.24 g of KH₂PO₄, dissolved in 1 L of double-distilled water) and ultrasonically disrupted. Then the supernatant was collected by centrifugation and loaded on the protein purification column for polyhistidine-tagged protein of protein purification system. And then a desired protein was eluted with the elution buffer for protein purification system (50 ml of glycerol, 8 g of NaCl, 0.201 g of KCl, 1.44 g of Na₂HPO₄, 0.24 g of KH₂PO₄, 17 g of imidazole, dissolved in 1L of double-distilled water). The protein concentration could be determined by a spectrophotometer or BCA Protein Assay Kit. Each purified fusion protein was aliquoted and stored at -20°C. The SDS-PAGE results of each protein were shown in FIGS. 3 to 4.

### 1.3 Construction of BEK293T-GFPβ11 cell line

### 1.3.1 Construction of lentiviral plasmid for GFPβ11 cell line:

The coding sequence of Histone-H3 (SEQ ID NO: 25) was obtained by PCR amplification, the coding sequence of GFPβ11 (SEQ ID NO: 26) was shorter and directly designed in the forward primer. The components were ligated through multiple rounds of PCR, and in the last round of PCR, the *Hind III* restriction site and the overlap on upstream of the corresponding *Hind III* restriction site on Lenti vector were introduced at the 5' end of the fragment through the forward primer, and the *BamHI* restriction site and the overlap on downstream of the corresponding *BamHI* restriction site on Lenti vector were introduced at the 3' end of the fragment through the reverse primer. The Lenti plasmid was subjected to double digestion with *Hind III* and *BamHI.* The insert fragments with overlaps were ligated to the digested Lenti vector by GIBSON assembly.

### 1.3.2 Lentivirus packaging, infection and resistance screening of cell line:

HEK-293T cells were inoculated into a 6-well plate and cultured overnight, and it was ensured that the number of cells per well was about 2^{∗}10⁷/ml before plasmid transfection. Before transfection, the cells were transferred into serum-free DMEM medium. 1.5 µg of Lenti recombinant plasmid, 0.75 µg of pMDL plasmid, 0.45 µg of pVSV-G plasmid, 0.3 µg of pREV (mass ratio 5:3:2:1) were added to 300 µl of serum-free DMEM, followed by slowly blowing. 9 µl (1:3) of X-tremeGENE transfection reagent was added and slowly blown, and was set aside at room temperature for 15 minutes. The cell supernatant was added dropwise, and after 8 hours, the culturing was continued by changing the medium to DMEM containing 10% FBS. after 60 hours, the culture supernatant was collected for later infection.

HEK-293T cells were inoculated into a 12-well plate and cultured overnight, and it was ensured that the number of cells per well was about 2^{∗}10⁶/ml (50% density) before lentivirus infection. The original cell culture supernatant was discarded, followed by addition of 300 µl of lentivirus (moi=3) and 700 µl of 10% FBS DMEM, and polybrene was added at a concentration of 10 µg/ml. The cell plate was centrifuged at 2500 rpm for 30 min under aseptic conditions, and the culture was continued.

After 48 hours of lentivirus infection, the cells were passaged at a ratio of 1/3, and puromycin was added at a concentration of 2.5µg/ml for resistance screening. The positive cells obtained from the screening were cloned to obtain HEK-293T-Hitone-GFPβ11 monoclonal cell line.

### 1.4 Detection of endosome escape efficiency of delivery system-GFPβ1-10 complex by Split-GFP system

The HEK-293T-Hitone-GFPβ11 cell line obtained in 1.3 was inoculated into a 12-well plate and cultured overnight, and it was ensured that the number of cells per well was about 5^{∗}10⁶/ml before protein treatment. After rinsing the cells with serum-free DMEM medium three times, 100µl/5µM of the delivery system-GFPβ1-10 complex obtained in 1.2 was added in serum-free medium, and incubation was performed for 3 h. Heparin solution was used for washing three times to remove the protein that had been adsorbed on the cell surface and had not yet been endocytosed into the cell, and the culturing was continued after changing the medium to 10% FBS DMEM medium. The observation with fluorescence microscope and the flow cytometry analysis of the expression of green fluorescent protein were performed at 12 h.

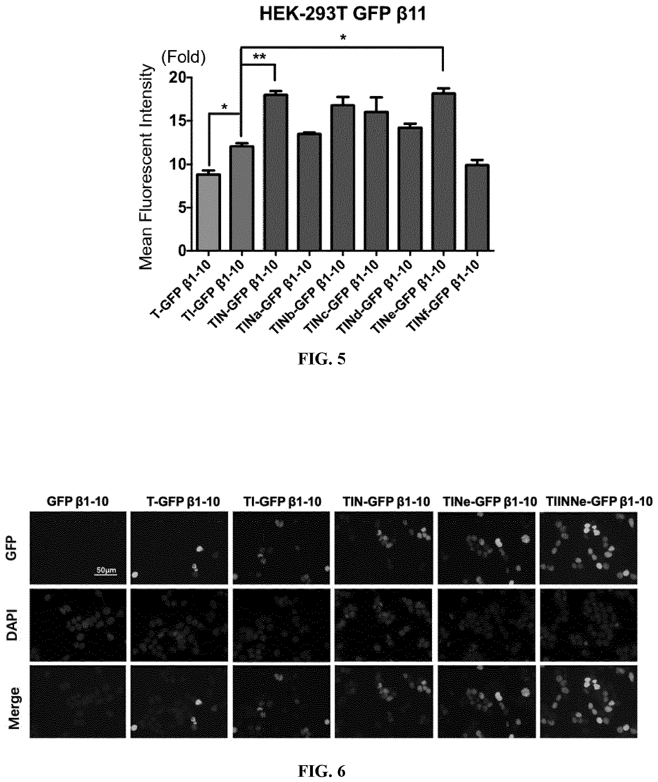

The results of flow cytometry analysis were shown in FIG. 5. The results showed that the average fluorescence intensity of the cells was increased when the pH-sensitive peptide INF7 was introduced on the basis of TAT, which proved that the pH-sensitive peptide had the effect of breaking endosome membrane. In particular, the cell fluorescence intensity was further increased when CTSL or Furin cleavage sites were introduced on the basis of TAT-INF7, in which the CTSL cleavage site N and the Furin cleavage site Ne showed the most significant effects. The fluorescence microscope observation was shown in FIG. 6, and the results showed that the combination of the above two cleavage sites (TINNe-GFP β1-10) could further significantly improve the endosome escape efficiency.

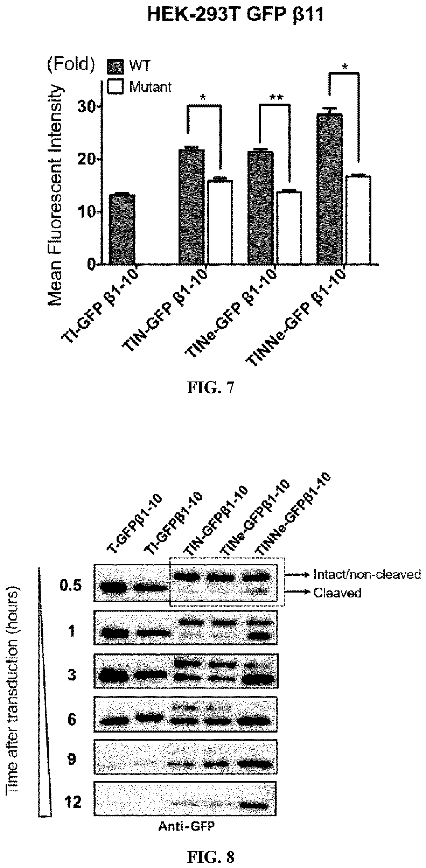

A mutation was introduced at a key site in the N or Ne cleavage site to obtain a delivery system-cargo molecule complex containing the mutation, wherein the mutant N had a sequence as shown in SEQ ID NO: 21, and the mutant Ne had a sequence as shown in SEQ ID NO: 22. The comparison of transfection efficiency between the mutant delivery system-cargo molecule complex (Mut) and the delivery system without mutation-cargo molecule complex (WT) was performed. The results were shown in FIG. 7. When the key amino acid in the cleavage site was mutated, whether it was a single cleavage site alone or a combination of two cleavage sites, the effect of enhancing the endosome escape efficiency was lost. The above results indicated that CTSL and Furin cleavage sites could indeed significantly improve the escape efficiency of cargo from endosome.

After washing three times with heparin solution to remove the protein that was adsorbed on the cell surface and had not yet been endocytosed into the cell, the cells were collected at different time points after the start of transduction. The cells were lysed to extract proteins and SDS-PAGE electrophoresis was performed, and then the monoclonal antibody (Abcam, ab32146) that recognized GFPβ1-10 was used to perform Western blot detection so as to analyze the cleavage and retention of the intracellular proteins. The results were shown in FIG. 8. At each time point before 6 hours, the total amounts of the intracellular proteins detected were comparable among the cells of the different groups, indicating that the pH-sensitive peptide and cleavage sites did not increase the endocytosis efficiency of cargo; the cleavage of the proteins (TIN-, TINe-, TINNe-) containing cleavage sites began within 30 minutes, and the amount of cleaved proteins no longer increased after 3 hours; wherein, about 40% of the proteins with single cleavage site was cleaved, while about 70% of the proteins with double cleavage sites was cleaved; since then, the proteins (T-, TI-) without cleavage site and the intact proteins whose cleavage site was not cleaved were introduced to lysosomes and began to degrade rapidly, and the corresponding bands almost disappeared at 12 h, while the proteins separated from TAT-INF7 after cleavage were still remained, and the TINNe group remained the most. The above results were consistent with the results of flow cytometry, that was, the higher the efficiency of enzyme cleavage, the more undegraded GFPβ1-10-NLS remained in the cell, the more intact GFP formed after GFPβ1-10-NLS entered into the nucleus and associated with GFPβ11, and the higher green fluorescence intensity.

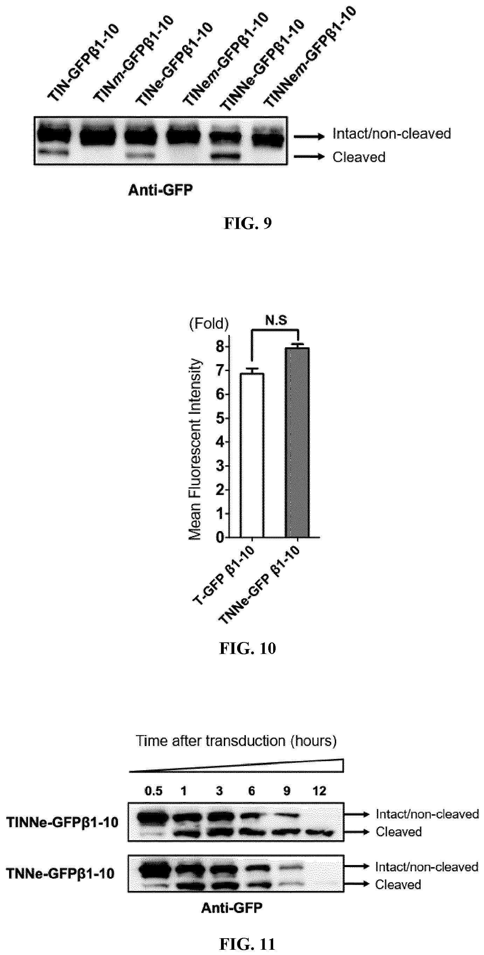

Further, Western blot was performed to analyze the cleavage of the above-mentioned delivery system-cargo molecule complex containing mutations in the N or Ne cleavage site after transduction of the cells for 3 h. The results were shown in FIG. 9. The intracellular cleavage of the mutant delivery system-cargo molecular complexes (TINm-, TINNem- and TINNem-) could not be fulfilled. Therefore, combined with the flow cytometry results shown in FIG. 7, it could be seen that the reason of low delivery efficiency of the mutant delivery system-cargo molecule complexes was that the cleavage and the subsequent escape could not be could not be achieved. It also further confirmed that CTSL and Furin cleavage sites played a key role in the delivery system.

In addition, the delivery efficiency of the delivery system-complex (TNNe-GFPβ1-10-NLS) lacking the pH-sensitive peptide component was also detected by flow cytometry, and the results were shown in FIG. 10. We found that the average fluorescence intensity after 12 h of transduction was basically the same as that of T-GFPβ1-10-NLS, indicating that even if there was a cleavage site, the lack of pH-sensitive peptide could not achieve efficient delivery. And it was found by Western blot that the presence or absence of pH-sensitive peptide had no effect on the intracellular cleavage efficiency of the delivery system-complex (FIG. 11). Combining the above results, we confirmed that only the coexistence of pH-sensitive peptide and specific cleavage site in the delivery system could achieve the final high-efficiency delivery.

### Example 2: Application of delivery system in transduction of zinc finger protein ZFP

### 2.1 Construction of expression vector for delivery system-zinc finger protein ZFP9 complex

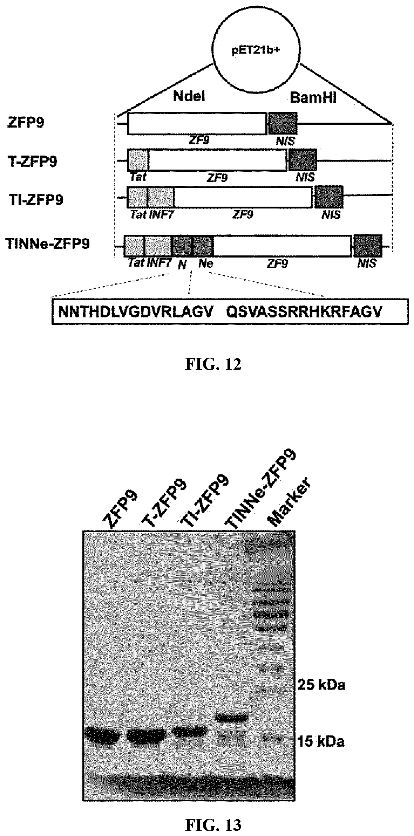

An expression vector of a recombinant protein containing TAT, INF7, protease cleavage site, and cargo molecule ZFP9 carrying nuclear localization signal (NLS) (SEQ ID NO: 27) was constructed, the schematic diagram of the structure of each recombinant protein was shown in FIG. 12, and the amino acid sequences of the components were shown in Table 4 below. The construction method was as follows: first, the nucleic acid sequences encoding the TAT, INF7, protease cleavage site, and cargo molecule ZFP9 in the delivery system were obtained by PCR amplification, and the parts were ligated by multiple rounds of PCR, and in the last round of PCR process, the *NdeI* restriction site and the overlap on upstream of the corresponding *NdeI* restriction site on pET-21b(+) were introduced at the 5' end of the fragment through the forward primer, and the *BamHI*restriciton site and the overlap on downstream of the corresponding *BamHI* restriction site on pET-21b(+) were introduced at the 3' end of the fragment through the reverse primer. The pET-21b(+) plasmid was subj ected to double digestion with *NdeI* and *BamHI.* The insert fragments with overlaps were ligated to the digested vector pET-21b(+) by GIBSON assembly.

**Table 4: Components contained in delivery system-cargo molecular complexes**

| Complex name | components from N-terminal to C-terminal and their sequences | | | |
|---|---|---|---|---|
| | CPP | pH-sensitive peptide | Protease cleavage sequence | Cargo |
| ZFP9 | None | None | None | ZFP9-NLS SEQ ID NO: 27 |
| T-ZFP9 | Tat SEQ ID NO: 10 | None | None | |
| TI- ZFP9 | | INF7 SEQ ID NO: 8 | | |
| TINNe-ZFP9 | | | N+Ne | |

### 2.2 Expression and purification of delivery system-ZFP9 complex

The expression plasmid described in 2.1 was transformed into the expression strain *E. coli* BL21 (DE3). A single colony was picked from the plate after transformation and inoculated in 5 ml of LB liquid medium containing ampicillin resistance and cultivated overnight, and then 1 ml of the overnight-cultured bacterial culture was transferred to 500 ml of LB liquid medium containing ampicillin resistance, followed by cultivation at 37°C and 180 rpm until the bacterial culture had an OD₆₀₀ of about 0.6, and then inducer IPTG was added to a final concentration of 0.2 mM, followed by induction at 25°C for 8 hours. After the induction, the bacterial cells were collected after centrifugation at 7000 g for 10 min at 4°C, and part of the bacterial cells were taken to detect the induction expression of protein. Then the bacterial cells were re-suspended with 10 ml of equilibration buffer for protein purification (50 ml of glycerol, 3.6342 g of Tris(hydroxymethyl)aminomethane, dissolved in 1 L of double-distilled water, adjusted pH to 8.0), and disrupted with ultrasonic. Then, the supernatant was collected by centrifugation and load on the Sulphopropyl (SP) cation exchange column of AKTA protein purification system; then desired proteins were obtained by gradient elution with the equilibration buffer and high-salt eluent (50 ml of glycerol, 116.88 g of NaCl, 3.6342 g of Tris(hydroxymethyl)aminomethane, dissolved in 1 L of double-distilled water, and adjusted pH to 8.0) in different proportions. The protein concentration could be determined according to spectrophotometer or BCA Protein Assay Kit. Each purified fusion protein was aliquoted and stored at -20°C. The SDS-PAGE results of each protein were shown in FIG. 13.

### 2.3 Construction of eukaryotic expression plasmid containing ZFP9 binding site

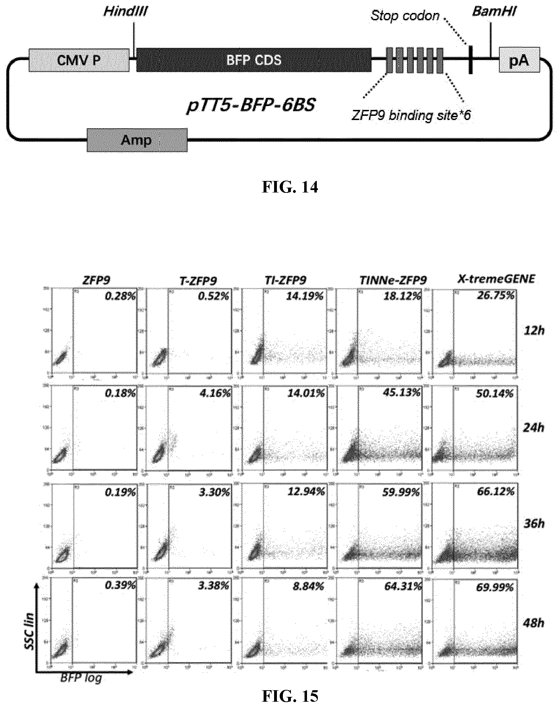

An expression vector containing the coding sequence of blue fluorescent protein (BFP) and ZFP9 binding sites was constructed, the structure schematic diagram of which was shown in FIG. 14. The coding sequence of blue fluorescent protein (SEQ ID NO: 29) and the sequence of ZFP9 binding sites (6^{∗} binding sites) (SEQ ID NO: 30) were obtained by PCR amplification, and the two parts were ligated by two rounds of PCR; and in the second round of PCR, the *Hind III* restriction site and the overlap on upstream of the corresponding *Hind III*restriction site on pTT5 vector were introduced at the 5' end of the fragment through the forward primer, and the BamHI restriction site and the overlap on downstream of the corresponding *BamHI* restriction site on pTT5 vector were introduced at the 3' end of the fragment through the reverse primer. The pTT5 plasmid was double-digested with *Hind III* and *BamH I.* The insert fragments with overlaps were ligated to the digested pTT5 vector by GIBSON assembly to obtain pTT5-BFP-6BS plasmid.

### 2.4 Detection of delivery efficiency of transducing zinc finger protein ZFP9 by delivery system

HEK293T cells were inoculated in a 12-well plate and cultured overnight, and it was ensured that the number of cells in each well was about 5^{∗}10⁶/ml before protein treatment. 100 µL/5 µM of the delivery system-ZFP9 complex (ZFP9, T-ZFP9, TI-ZFP9, TINNe-ZFP9) obtained in 2.2 and 5 µg of the pTT5-BFP-6BS plasmid obtained in 2.3 were co-incubated at 37°C for 30 minutes to fully form a complex, and X-tremeGENE transfection reagent (Roche) was used as a positive control (5 µg of plasmid and 15 µl of transfection reagent were mixed and used to transfect cells under serum-free conditions, and after 8 hours, the culturing was continued by changing the medium to 10% FBS DMEM), The cells were rinsed with serum-free DMEM medium three times, and then the complex was added and incubated for 3h. Heparin solution was used to perform washing three times to remove the protein that was adsorbed on the cell surface and had not yet been endocytosed into the cell, then the culturing was continued after changing the medium to 10% FBS DMEM medium, and the flow cytometry analysis of blue fluorescent protein expression was performed at 12h, 24 h, 36 h, and 48 h after the changing of medium.

The results of the flow cytometry analysis were shown in FIGS. 15 to 16. The plasmid bound to ZFP9 could complete the transcription process only when it entered the nucleus. Therefore, only when ZFP9 escaped from endosome, it could carry the bound plasmid to enter into the nucleus, and the transcription process could thus be started to express blue fluorescent protein. Compared with T-ZFP9, the introduction of pH-sensitive peptide INF7 (TI-ZFP9) could increase the blue fluorescence ratio by about 10% (p=0.035), but it was still at a low level, that was, most of the complex failed to escape and enter into the nucleus. While further introduction of a protease cleavage site into the system (TINNE-ZFP9), could significantly increase the proportion of blue fluorescent cells, reaching about 65% at 48h (p=0.018), which was be equal to that of the Roche transfection reagent X-tremeGENE. It could be seen that during the endocytosis process of the delivery system carrying the ZFP9/pTT5-BFP-6BS complex into the cell, the introduction of CTSL and Furin-specific cleavage sites N and Ne significantly promoted the escape process of the carried ZFP9, more of the complex could enter into the nucleus to complete transcription, and then expressed BFP fluorescence.

Example 3: Application of delivery system in transduction of protein phosphatase Ppm1b TNF-α binds to cell surface receptors to induce RIP3 phosphorylation and form necrosomes of multi-protein complexes, and the phosphorylated RIP3 in the necrosomes recruits and phosphorylates Mlkl and then the cells enter the necrosis program. In this process, the intracellular protein phosphatase 1B (Ppm1b) can inhibit programmed cell necrosis (Necroptosis) by dephosphorylating RIP3. In view of the fact that programmed cell necrosis has been found to be closely related to the occurrence of inflammatory diseases, ischemia-reperfusion injury, neurodegenerative diseases and other diseases, Ppm1b protein has shown great potential in the treatment of the above-mentioned diseases related to programmed cell necrosis.

### 3.1 Construction of expression vector for delivery system-Ppmlb protein complex

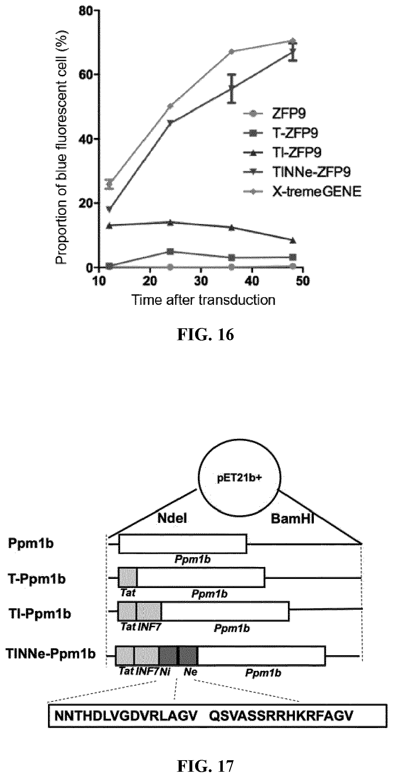

An expression vector for a recombinant protein containing TAT, INF7, protease cleavage site, and cargo molecule Ppm1b (SEQ ID NO: 31) was constructed. The structure diagram of each recombinant protein was shown in FIG. 17, and the amino acid sequence of each component was shown in Table 6 below. The construction method was as follows: the nucleic acid sequences of TAT, INF7, protease cleavage site, and Ppm1b were obtained by PCR amplification, and the parts were ligated by multiple rounds of PCR, and in the last round of PCR, the *NdeI* restriction site and the overlap on upstream of the corresponding *NdeI* restriction site on pET-21b(+) were introduced at the 5' end of the fragment by forward primer, and the *BamHI*restriction site and the overlap on downstream of the corresponding *BamHI*restriction site on pET-21b(+) were introduced at the 3' end of the fragment through the reverse primer. The pET-21b(+) plasmid was subjected to double digestion with *NdeI* and *BamHI.* The insert fragments with overlaps were ligated to the digested vector pET-21b(+) by GIBSON assembly.

**Table 5: Components contained in delivery system-cargo molecular complexes**

| Complex name | components from N-terminal to C-terminal and their sequences | | | |
|---|---|---|---|---|
| | CPP | pH-sensitive peptide | Protease recognition sequence | Cargo |
| Ppm1b | None | None | None | Ppm1b SEQ ID NO: 31 |
| T- Ppm1b | Tat SEQ ID NO:10 | None | None | |
| TI- Ppm1b | | INF7 SEQ ID NO: 8 | | |
| TINNe- Ppm1b | | | N+Ne | |

### 3.2 Expression and purification of delivery system-Ppmlb complex

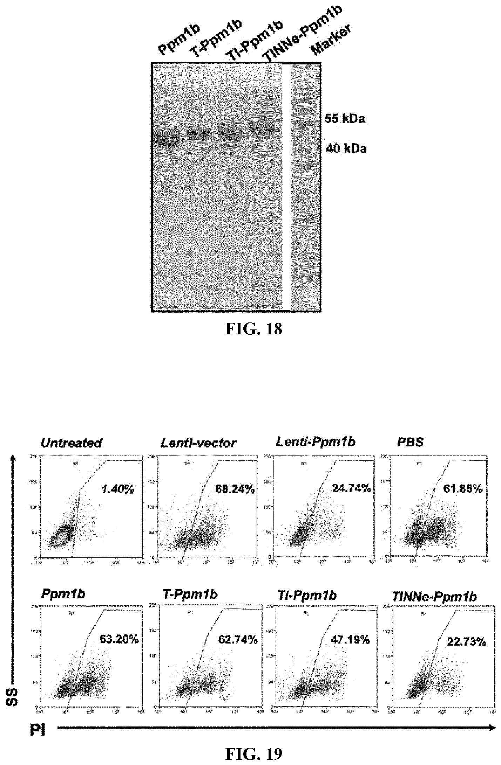

The expression plasmid described in 3.1 was transformed into the expression strain *E. coli* BL21 (DE3). a single colony was picked from the plate after transformation and inoculated in 5 ml of LB liquid medium containing ampicillin resistance and cultivated overnight, and then 1 ml of the overnight-cultured bacterial culture was transferred into 500ml of LB liquid medium containing ampicillin resistance, followed by cultivation at 37°C and 180 rpm until the bacterial culture had an OD₆₀₀ of about 0.6, and then inducer IPTG was added to reach a final concentration of 0.2 mM, followed by induction at 25°C for 8 h. After the induction, the bacterial cells were collected by centrifugation at 7000 g for 10 minutes at 4°C, and a part of the bacterial cells was taken to detect the induction expression of protein. Then the bacterial cells were re-suspended in 10ml of equilibration buffer for protein purification (50 ml of C₃H₈O₃, 3.6342 g of Tris(hydroxymethyl)aminomethane, dissolved in 1 L of double-distilled water, adjusted pH to 8.0), and disrupted by ultrasonic. Then the supernatant was collected by centrifugation and loaded on the Sulphopropyl (SP) cation exchange column of AKTA protein purification system; then the desired proteins were obtained by gradient elution with the equilibration buffer and high-salt eluent (50 ml of C₃H₈O₃, 116.88 g of NaCl, 3.6342 g of Tris(hydroxymethyl)aminomethane, dissolved in 1L of double-distilled water, adjusted to pH 8.0) in different proportions. The protein concentration could be determined by spectrophotometer or BCA Protein Assay Kit. Each purified fusion protein was aliquoted and stored at -20°C. The SDS-PAGE results of each protein were shown in FIG. 18.

### 3.3 Effect of delivery system-Ppmlb complex on rate of necrosis induced by TNF-α

L929 cells were inoculated in a 12-well cell culture plate and cultured overnight, and it was ensured that the number of cells per well was about 2^{∗}10⁶/ml before protein treatment. The cells were rinsed with serum-free DMEM medium three times, and then 100µl/5µM of the delivery system proteins (Ppm1b, T-Ppm1b, TI-Ppm1b, TINNe-Ppm1b) obtained in 3.2 were added in serum-free medium respectively, and incubated for 3 hours. Then 1 ml of 10% FBS DMEM containing 20 ng/ml TNF-α and 20mM z-VAD was added, and incubated for 10h. The cells were collected and subjected to PI staining, and the flow cytometry analysis was performed to observe the ratio of cell necrosis. Using lentivirus-transduced Ppm1b (Lenti-Ppm1b) and lentivirus (Lentivec) as controls, the lentivirus with Ppm1b expression sequence and the control lentivirus were packaged and collected on HEK-293T cells, and after infecting L929 cells for 24 h to make Ppm1b fully expressed in the cells, the infected L929 cells were re-plated for later TNF-α stimulation.

The results were shown in FIG. 19. Compared with T-Ppm1b, the introduction of pH-sensitive peptide INF7 (TI-Ppm1b) could merely slightly inhibit cell necrosis, but it was still at a low level. However, by further introduction of the protease cleavage site in the system (TINNe-Ppm1b), the TINNe group had an ability of inhibiting cell necrosis that was comparable to the level of the lentivirus group, and the cell necrosis rate can be greatly reduced to about 25%. Therefore, during the endocytosis process of the delivery system carrying the Ppm1b complex into the cell, the introduction of CTSL and Furin specific sites N and Ne significantly promoted the escape process of the carried Ppm1b, and more Ppm1b could enter into the cytoplasm and inhibit the phosphorylation process of RIP3, thereby reducing the rate of cell necrosis.

### Example 4: Application of delivery system in gene-editing enzyme Cas9

In the CRISPR/Cas9 gene editing system, sgRNA binds to Cas9 protein, and sgRNA can specifically recognize a target site and Cas9 can bind and cut DNA double-stranded molecule, and the editing of a targeted gene is realized through non-homologous end recombination or homology-directed repair. In this system, Cas9 must enter the nucleus to complete its function. Based on this, we fused the delivery system with the Cas9 protein so as to achieve the gene editing on eukaryotic cells.

### 4.1 Construction of expression vector for delivery system-Cas9 protein complex

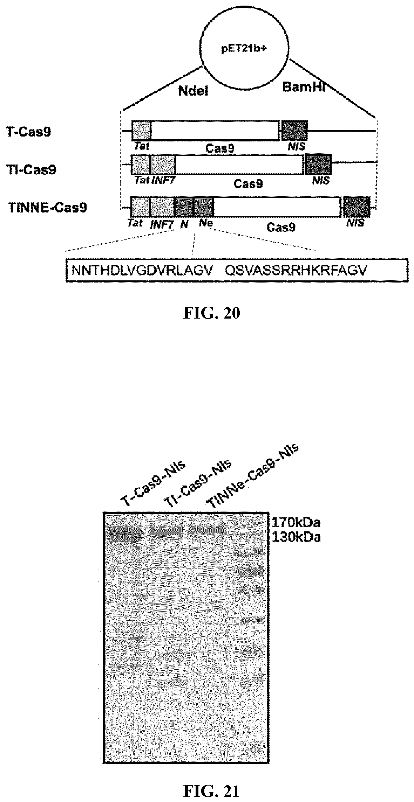

An expression vector for a recombinant protein containing TAT, INF7, protease cleavage site, and cargo molecule Cas9 carrying nuclear localization signal (NLS) (SEQ ID NO: 33) was constructed. The schematic diagram of the structure of each recombinant protein was shown in FIG. 20, and the amino acid sequence of each component was shown in Table 6 below. The construction method was as follows: the nucleic acid sequences encoding TAT, INF7, protease cleavage site N and Ne, Cas9 were obtained by PCR amplification, and the parts were ligated by multiple rounds of PCR; in the last round of PCR, the *NdeI* restriction site and the overlap on upstream of the corresponding *NdeI* restriction site on pET-21b(+) were introduced at the 5' end of the fragment through the forward primer, and the *BamHI* restriction site and the overlap on downstream of the corresponding *BamHI* restriction site on pET-21b(+) were introduced at the 3' end of the fragment through the reverse primer. The pET-21b(+) plasmid was subjected to double digestion with *NdeI* and *BamHI.* The insert fragments with overlaps were ligated to the digested vector pET-21b(+) by GIBSON assembly.

**Table 6: Components contained in delivery system-cargo molecular complexes**

| Complex name | components from N-terminal to C-terminal and their sequences | | | |
|---|---|---|---|---|
| | CPP | pH-sensitive peptide | Protease recognition sequence | Cargo |
| T- Cas9 | Tat SEQ ID NO:10 | None | None | Cas9-NLS SEQ ID NO: 33 |
| TI- Cas9 | | INF7 SEQ ID NO: 8 | | |
| TINNe- Cas9 | | | N+Ne | |

### 4.2 Expression and purification of delivery system-Cas9 complex

Preliminary purification on nickel column: The expression plasmid described in 4.1 was transformed into the expression strain *E. coli* BL21 (DE3); a single colony was picked from the plate after transformation and inoculated into 5ml of LB liquid medium containing ampicillin resistance and cultivated overnight; then 1 ml of the overnight-cultured bacterial culture was transferred into 500 ml of LB liquid medium containing ampicillin resistance, cultured at 37°C and 180 rpm until the bacterial culture had an OD₆₀₀ of about 0.6; then the inducer IPTG was added to reach a final concentration of 0.2 mM, followed by induction for 8 h at 25°C; after the induction, the bacterial cells were collected by centrifugation at 7000 g and 4°C for 10 minutes; then the bacterial cells were re-suspended with 10ml of equilibration buffer for protein purification (5% glycerol, 30mM TB8.0, 50mM glycerol, 500mM sodium chloride, 25mM glucose) and disrupted with ultrasonic. Then the supernatant was collected by centrifugation and loaded on the protein purification column for polyhistidine-tagged protein of protein purification system; then the desired protein was eluted by protein purification system with the elution buffer for protein purification system (5% glycerol, 30 mM TB8.0, 50 mM glycerol, 500 mM sodium chloride, 25 mM glucose, 250 mM imidazole).

Final purification on cation exchange column: The desired protein collected from the preliminary purification on nickel column was dialysed into an equilibrium buffer (30 mM TB8.0, 50 mM glycerol, 250 mM sodium chloride, 25 mM glucose, adjusted pH to 7.2), loaded on the Sulphopropyl (SP) cation exchange column of AKTA protein purification system; then the desired protein was obtained by gradient elution with the equilibration buffer and high-salt eluent (30 mM TB8.0, 50 mM glycerol, 2 M sodium chloride, 25 mM Glucose, adjusted pH to 7.2) in different proportions. The protein concentration can be measured by spectrophotometer or BCA Protein Assay Kit. Each purified fusion protein was aliquoted and stored at -20°C. The SDS-PAGE results of each protein were shown in FIG. 21.

### 4.3 Construction of HEK293T-RFP reporter cell line

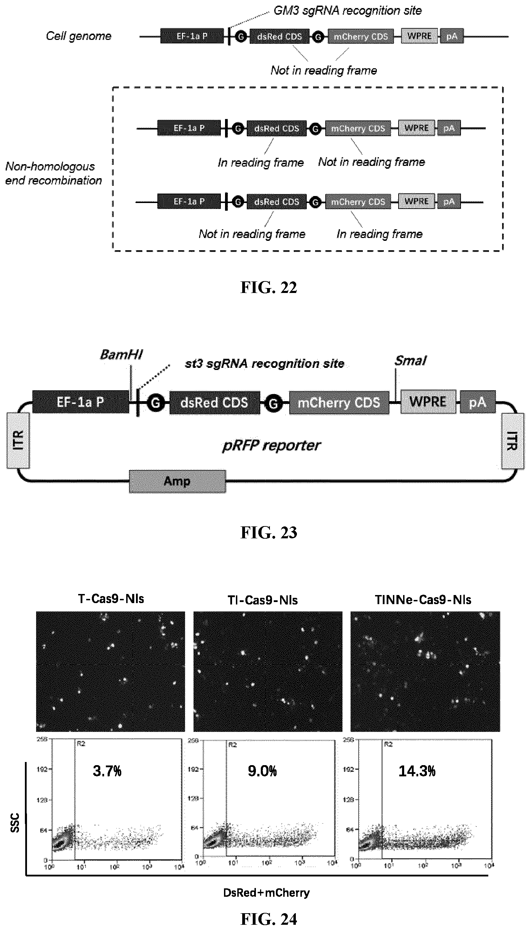

Cas9 can specifically recognize a target site by binding to sgRNA, and non-homologous end recombination will occur if a donor is not provided. Therefore, the sgRNA recognition site and two red fluorescent protein genes that are not in a reading frame (dsRed and mCherry are linked by G) are integrated into the genome of HEK-293T cells by means of lentivirus infection. In such case, if Cas9 causes the occurrence of homologous end recombination of DNA sequence on the sgRNA recognition site, it will cause the red fluorescent protein gene that is not in the reading frame to enter the reading frame and be expressed, which will change the cell from non-fluorescent to red fluorescent state, so that the efficiency of the delivery system for transduction of genetically engineered enzyme Cas9 can be evaluated by whether red fluorescence is produced and the number of red fluorescent cells (FIG. 22).

### 4.3.1 Construction of lentiviral plasmid for RFP repoter cell line

The coding sequence of dsRed (SEQ ID NO: 35) and the coding sequence of mCherry (SEQ ID NO: 36) were obtained by PCR amplification, the DNA sequence of sgRNA recognition site (SED ID NO: 37) was shorter and thus designed in the primer, and the components were ligated to by multiple rounds of PCR. In the last round of PCR, the *Hind III* restriction site and the overlap on upstream of the corresponding *Hind III* restriction site on Lenti vector were introduced at the 5' end of the fragment through the forward primer, and the *BamHI* restriction site and the overlap on downstream of the corresponding *BamHI*restriction site on Lenti vector were introduced at the 3' end of the fragment through the reverse primer. The Lenti plasmid was subjected to double digestion with *Hind III* and *BamHI.* The insert fragments with overlaps were ligated to the digested Lenti vector by GIBSON assembly. The map of successfully constructed plasmid is shown in FIG. 23.

### 4.3.2 Lentivirus packaging, infection and cell line resistance screening

HEK293T cells were inoculated in a 6-well plate and cultured overnight, and it was ensured that the number of cells per well was about 2^{∗}10⁷/ml before plasmid transfection. Before transfection, the medium was replaced with serum-free DMEM medium. 1.5 µg of Lenti recombinant plasmid (RFP reporter), 0.75 µg of pMDL plasmid, 0.45 µg of pVSV-G plasmid, 0.3 µg of pREV plasmid (the mass ratio was 5:3:2:1) were added in 300 µl of serum-free DMEM, slowly blown well, and allowed to stand for 5 minutes. Then 9 µl of X-tremeGENE transfection reagent was added, slowly blown well, and allowed to stand at room temperature for 15 minutes. Then it was added to the cell supernatant. 8 hours later, the culturing was continued after changing the medium to DMEM containing 10% FBS. 60 hours later, the culture supernatant was collected and stored at 4°C.

HEK293T cells were inoculated into a 12-well plate and cultured overnight, and it was ensured that the number of cells per well was about 2^{∗}10⁶/ml (50% density) before lentivirus infection. The cell culture supernatant was discarded, and 300 µl of lentivirus (Moi=3), 700 µl of 10% FBS DMEM and polybrene at a concentration of 10 µg/ml were added, followed by centrifugation at 2500 rpm for 30 minutes under aseptic conditions, and then the culturing was continued.

After 48 hours of lentivirus infection, the cells were passaged at a ratio of 1/3, and puromycin at a concentration of 2.5 µg/ml was added to perform resistance screening; the positive cells obtained by the screening were cloned to obtain monoclonal cell strain of HEK293T-RFP reporter.

### 4.3.3 Construction of GM3-gRNA transcription plasmid

The introduction of gRNA is performed by transfection of a transcription plasmid which will be transcribed into gRNA in the cell. The DNA sequence corresponding to the gRNA is generated by primer annealing and overlapping. In the process of primer design, the sticky end of the *AflII* restriction site was directly introduced; gRNA-cloning vector was treated with *AflII* single enzyme digestion; the vector and insert fragment were ligated by T4 DNA ligase using their respective sticky ends.

### 4.4 Evaluation of editing efficiency of delivery system-Cas9 complex

Transfection of gRNA transcription plasmid: the HEK-293T-RFP reporter cell line was inoculated into a 12-well plate and cultured overnight, and it was ensured that the number of cells per well was about 2.5^{∗}10⁶/ml before transfection. Before transfection, the medium was replaced with serum-free DMEM. 1 µg of gRNA-GM3 transcription plasmid was added to 100 µl of serum-free DMEM, slowly blown well, allowed to stand for 5 min. 3 µl of X-tremeGENE transfection reagent was further added, blown well, and allowed to stand for 15 min. It was then added to the cell supernatant. After 8 hours, the medium was changed to 10% FBS DMEM.

Transduction of Cas9 by delivery system: 12 h after the transfection of gRNA transcription plasmid (4 h after changing to serum-containing DMEM), the cells were rinsed three times with serum-free DMEM, and 5 µM of the delivery system-Cas9 complex obtained in 4.2 was added under serum-free DMEM condition and incubated for 3 h. The medium was changed to 10% FBS DMEM and the culturing was continued, and the observation and flow cytometry analysis of red fluorescence protein expression were performed at 48 h.

The flow cytometry results were shown in FIG. 24. After transduction of T-Cas9, the proportion of red fluorescent cells was low (3.7%), that was, most of the Cas9 protein failed to escape and enter into the nucleus; the introduction of pH-sensitive peptide INF7 (TI-Cas9) could increase the proportion of red fluorescent cells to 9%; and the further introduction of a protease cleavage site into the system (TINNE-Cas9), significantly increased the number of cells expressing red fluorescent protein, which could reach about 14% in 48 h. Therefore, during the endocytosis process of the delivery system carrying Cas9 into the cell, the introduction of CTSL and Furin specific sites N and Ne significantly promoted the escape process of the carried Cas9, and more Cas9 entered into the nucleus under the action of the nuclear localization signal. After binding to the GM3 sgRNA formed by intracellular transcription, Cas9 specifically recognized the target sequence, and homologous end repair would occur near this site, so that the red fluorescent gene entered the reading frame and was expressed.

### Example 5: Establishment of delivery system based on non-covalent connection

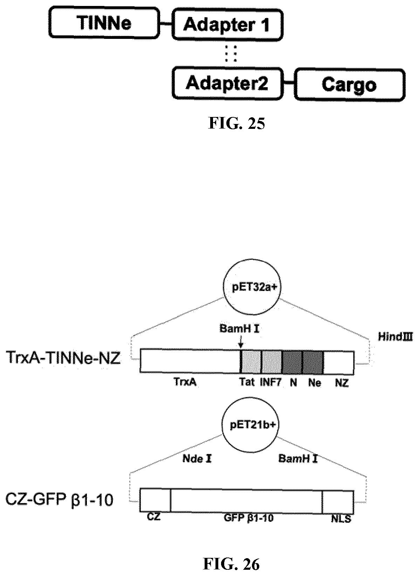

In addition to fusion expression, the linking mode between the fusion protein of the present invention and cargo could also be non-covalent interaction through protein domains, which called adapter pairs, such as heterodimer leucine zipper with strong interaction (FIG. 25). Two antiparallel leucine zipper domains can spontaneously combine to form an oligomer due to the complementarity in their spatial structure and charge distribution. It is reported that, NZ and CZ are such a pair of leucine zippers that can be combined with each other. We fused the NZ domain to the intracellular delivery system of the present invention (TINNe-NZ), and fused CZ domain to GFPβ1-10 with nuclear localization signal (NLS) as Cargo (CZ-GFPβ1-10-NLS). The two fused proteins were mixed and incubated to combine with each other, and were subjected to transduction into the HEK293T cells stably expressing Histone-β11, and the endosome escape efficiency could be evaluated by the proportion of GFP and the relative fluorescence intensity. Therefore, the feasibility of using the adapter pair of NZ-CZ to link the fusion protein to the cargo was evaluated.

### 5.1 Construction of expression vector for delivery system-NZ fusion protein and CZ-GFPβ1-10 fusion protein

Based on the pET32a vector, the expression vector for recombinant protein containing TAT, INF7, protease cleavage site and NZ domain, and the expression vector for recombinant protein containing CZ domain, cargo molecule GFPβ1-10 with nuclear localization signal (NLS), were constructed, in which TrxA was introduced as a solubilizing label. The structural schematic diagram of each recombinant protein was shown in FIG. 26, and the amino acid sequence of the delivery system was shown in the following table. The construction method was as follows: first, the aforementioned vector was used as a template, the nucleic acid sequences encoding TAT, INF7, N, Ne, and NZ sequences in the delivery system, as well as the CZ sequence and the cargo molecule sGFPβ1-10, were obtained by PCR amplification. For TrxA-TINNe-NZ, in the last round of PCR process, the *BamHI* restriction site and the overlap on upstream of the corresponding *BamHI* restriction site on pET-32a(+) were introduced at the 5' end of the fragment through the forward primer, the *HIindIII* restriction site and the overlap on downstream of the corresponding *HIindIII* restriction site on pET-32a(+) were introduced at the 3' end of the fragment through the reverse primer. The pET-32a(+) plasmid was subjected to double digestion with *BamHI* and *HIindIII.* The insert fragments with overlaps were ligated to the digested vector pET-32a(+) by GIBSON assembly. For CZ-GFP β1-10, in the last round of PCR process, the *NdeI* restriction site and the overlap on upstream of the corresponding *NdeI* restriction site on pET21b(+) were introduced at the 5' end of the fragment through the forward primer, and the *BamHI* restriction site and the overlap on downstream of the corresponding *BamHI* restriction site on pET-21b(+) were introduced at the 3' end of the fragment through the reverse primer. The pET-21b(+) plasmid was subjected to double digestion with *NdeI* and *BamHI.* The insert fragments with overlaps were ligated to the digested vector pET-21b(+) by GIBSON assembly.

**Table 7: Components contained in delivery system**

| Delivery system | components from N-terminal to C-terminal and their sequences | | | | |
|---|---|---|---|---|---|
| | Solubilizing tag | CPP | pH-sensitive peptide | Protease recognition sequence | Specific binding sequence |
| TINNe-NZ | TrxA SEQ ID NO:53 | Tat SEQ ID NO:10 | INF7 SEQ ID NO: 8 | N+Ne | NZ SEQ ID NO:49 |

### 5.2 Expression and purification of delivery system-NZ fusion protein and CZ-GFPβ1-10 fusion protein

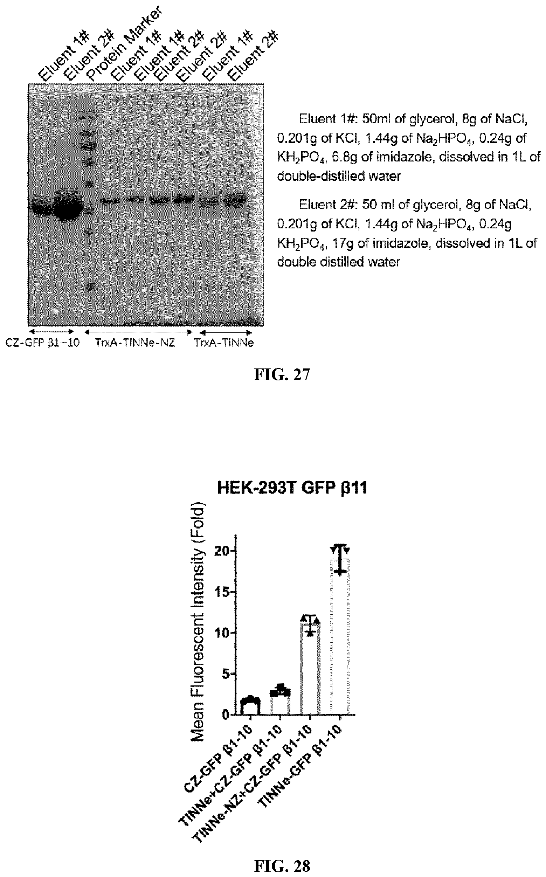

The expression plasmid described in 5.1 was transformed into the expression strain *E. coli* BL21 (DE3); a single colony was picked from the plate after transformation and inoculated in 5 ml of LB liquid medium containing ampicillin resistance and cultivated overnight. 1 ml of the overnight-cultured bacterial culture was transferred to 500ml of LB liquid medium containing ampicillin resistance, cultured at 37°C and 180 rpm until the bacterial culture had an OD₆₀₀ of about 0.6. Then the inducer IPTG was added to reach a final concentration of 0.2 mM for induction at 25°C for 8 h. After the induction, the bacterial cells were collected by centrifugation at 7000 g for 10 min at 4°C; then the bacterial cells were re-suspended in 10 ml of equilibration buffer for protein purification (50 ml of glycerol, 8 g of NaCl, 0.201 g of KCl, 1.44 g of Na₂HPO₄, 0.24 g of KH₂PO₄, dissolved in 1 L of double distilled water) and ultrasonically disrupted. The supernatant was collected by centrifugation and was loaded on the protein purification column for polyhistidine-tagged protein of protein purification system; then the desired protein was eluted by the protein purification system using the elution buffer for protein purification, Elution buffer 2# (50 ml of glycerol, 8 g of NaCl, 0.201 g of KCl, 1.44 g of Na₂HPO₄, 0.24 g of KH₂PO₄, 17 g of imidazole, dissolved in 1L of double-distilled water). The protein concentration could be determined by spectrophotometer or BCA Protein Assay Kit. Each purified fusion protein was aliquoted and stored at -20°C. The SDS-PAGE results of each protein were shown in FIG. 27.

### 5.3 Detection of delivery efficiency of adapter-based delivery system by Split-GFP system

The HEK-293T-Hitone-GFPβ11 cell line obtained above was inoculated into a 12-well plate and cultured overnight, and it was ensured that the number of cells per well was about 5^{∗}10⁶/ml before protein treatment; then the cells were rinsed with serum-free DMEM medium three times. The control groups and the experimental group and the amount of protein (obtained in 5.2) they used, were shown in the following table, in which before incubating with the cells, the two proteins in the Control Group 2 and the experimental group were mixed in a serum-free medium at room temperature for 10min. After incubation in a serum-free medium condition for 3 hours, the cells were washed three times with heparin solution to remove the protein that was adsorbed on the cell surface and had not yet been endocytosed, then the culturing was continued after changing the medium to 10% FBS DMEM medium, and the fluorescence microscope observation and the flow cytometry analysis of the proportion of green fluorescence positive cells and MFI were performed at 12 hours.

The flow cytometry analysis results were shown in FIG. 28. The results showed that the MFI values of the two control groups (CZ-GFPβ1~10/TrxA-TINNe+CZ-GFPβ1~10) were both lower than 5, in which the Control Group 2 (TrxA-TINNe+CZ-GFPβ1~10) had MFI slightly higher than that of the Control Group 1. We speculated that it was caused by the non-specific adsorption between TrxA-TINNe and CZ-GFPβ1~10, which enabled the cargo CZ-GFPβ1~10 to enter into the cell and escape from the endosome by TINNe. The MFI value of TrxA-TINNe-NZ+CZ-GFPβ1~10 group was close to 20, which was significantly higher than that of the control groups, which indicated that under the action of NZ and CZ, TrxA-TINNe combined with GFPβ1~10 in a certain degree, and through the action of TINNe, GFPβ1~10 entered into the cell and escaped from the endosome. Although the fluorescence intensity of the adapter-based linking method was still lower than that of the TINNe-GFPβ1~10 which was obtained by fusion expression, the experiment confirmed that the linking method based on NZ-CZ or similar adapter could be used to link the delivery system with the cargo, and could effectively deliver the cargo to enter into the cell and escape from endosome.

**Table 8: Control and experimental groups**

| Group | protein to be added and its final concentration | | | |
|---|---|---|---|---|
| | TrxA-TINNe | TrxA-TINNe-NZ | CZ-GFP β1-10 | TINNe-GFP β1-10 |
| Control Group 1 | | | 5µM | |
| Control Group 2 | 5µM | | 5µM | |
| Control Group 3 | | | | 5µM |
| Experimental Group | | 5µM | 5µM | |

Although the specific embodiments of the present invention have been described in detail, those skilled in the art will understand that various modifications and changes can be made to the details according to all the teachings that have been disclosed, and these changes are within the protection scope of the present invention. All of the present invention is given by the appended claims and any equivalents thereof.

## Claims

1. A fusion protein, which comprises a cell-penetrating peptide, a pH-sensitive fusogenic peptide and a protease recognition sequence, wherein the protease is selected from furin and/or lysosomal cysteine protease.

2. The fusion protein according to claim 1, wherein the furin recognition sequence comprises R-X₁-X₂-R (SEQ ID NO: 1), wherein X₁ is any amino acid, and X₂ is K or R;
preferably, the furin recognition sequence comprises R-R-X₁-X₂-R (SEQ ID NO: 2);
preferably, the furin recognition sequence comprises a sequence shown in SEQ ID NO: 3;
preferably, the furin recognition sequence comprises a sequence shown in SEQ ID NO: 4.

3. The fusion protein according to claim 1 or 2, wherein the lysosomal cysteine protease is selected from the group consisting of cathepsin B, cathepsin C, cathepsin X, cathepsin S, cathepsin L, cathepsin D or cathepsin H;
preferably, the lysosomal cysteine protease is cathepsin L;
preferably, the cathepsin L recognition sequence comprises a sequence shown in SEQ ID NO:6.

4. The fusion protein according to any one of claims 1 to 3, wherein the protease recognition sequence comprises a furin recognition sequence and a cathepsin L recognition sequence;
preferably, the protease recognition sequence comprises SEQ ID NO: 3 and SEQ ID NO: 6;
preferably, the protease recognition sequence comprises SEQ ID NO: 4 and SEQ ID NO: 6.

5. The fusion protein according to any one of claims 1 to 4, wherein the pH-sensitive fusogenic peptide is selected from influenza virus HA2 or its mutant (e.g., INF7, KALA or GALA), melittin, and any combination thereof;
preferably, the pH-sensitive fusogenic peptide comprises INF7;
preferably, the pH-sensitive fusogenic peptide comprises a sequence shown in SEQ ID NO: 8.

6. The fusion protein according to any one of claims 1 to 5, wherein the cell-penetrating peptide is selected from the group consisting of penetratin, Tat-derived peptide (e.g., Tat(48-60) or Tat(47- 57)), Rev(34-50), VP22, transportan, Pep-1, Pep-7, and any combination thereof;
preferably, the cell-penetrating peptide comprises a Tat-derived peptide, such as Tat(48-60);
preferably, the cell penetrating peptide comprises a sequence shown in SEQ ID NO: 10.

7. The fusion protein according to any one of claims 1 to 6, wherein the fusion protein comprises the pH-sensitive fusogenic peptide, cell penetrating peptide, and protease recognition sequence from N-terminus to C-terminus; or, the fusion protein comprises the cell-penetrating peptide, pH-sensitive fusogenic peptide and protease recognition sequence from N-terminus to C-terminus;
preferably, the protease recognition sequence comprises the furin recognition sequence and cathepsin L recognition sequence from N-terminus to C-terminus, or comprises the cathepsin L recognition sequence and furin-recognition sequence from N-terminus to C-terminus.

8. The fusion protein according to any one of claims 1 to 7, wherein the fusion protein comprises a sequence shown in any one of SEQ ID NOs: 12 to 14.

9. The fusion protein according to any one of claims 1 to 8, wherein the fusion protein further comprises a specific binding sequence, the specific binding sequence allows another molecule (e.g., polypeptide, protein, or nucleic acid) to specifically bind thereto;
preferably, the specific binding sequence comprises a leucine zipper peptide which is able to form a heterodimer with its complementary peptide;
preferably, the specific binding sequence comprises leucine zipper NZ or CZ;
preferably, the specific binding sequence comprises a sequence shown in SEQ ID NO: 49 or 50;
preferably, the specific binding sequence is located at the C-terminus of the protease recognition sequence.

10. A complex, which comprises the fusion protein according to any one of claims 1 to 9, and a cargo molecule;
preferably, the cargo molecule is selected from the group consisting of nucleic acid, peptide or protein, carbohydrate, lipid, chemical compound and any mixture thereof;
preferably, the nucleic acid is selected from the group consisting of DNA molecule, RNA molecule, siRNA, antisense oligonucleotide, ribozyme, aptamer and any combination thereof;
preferably, the cargo molecule comprises a detectable label;
preferably, the cargo molecule comprises an epitope tag, reporter gene sequence and/or nuclear localization signal (NLS) sequence.

11. The complex according to claim 10, wherein the fusion protein is fused with the cargo molecule, and the cargo molecule is a peptide or protein;
preferably, the cargo molecule is fused to the C-terminus of the fusion protein;
preferably, the complex comprises a single-chain polypeptide, and the single-chain polypeptide comprises the pH-sensitive fusogenic peptide, cell-penetrating peptide, protease recognition sequence and cargo molecule from N-terminus to C-terminus; preferably, the protease recognition sequence comprises the furin recognition sequence and cathepsin L recognition sequence from N-terminus to C-terminus, or comprises the cathepsin L recognition sequence and furin recognition sequence from N-terminus to C-terminus;
preferably, the complex comprises a single-chain polypeptide, and the single-chain polypeptide comprises the cell-penetrating peptide, pH-sensitive fusogenic peptide, protease recognition sequence and cargo molecule from N-terminus to C-terminus; preferably, the protease recognition sequence comprises the furin recognition sequence and cathepsin L recognition sequence from N-terminus to C-terminus, or comprises the cathepsin L recognition sequence and furin recognition sequence from N-terminus to C-terminus.

12. The complex according to claim 10, wherein the fusion protein is chemically coupled to the cargo molecule;
preferably, the chemical coupling is achieved through disulfide bond, phosphodiester bond, phosphorothioate bond, amide bond, amine bond, thioether bond, ether bond, ester bond or carbon-carbon bond;
preferably, the cargo molecule is coupled to the N-terminus or C-terminus of the fusion protein.

13. The complex according to claim 10, wherein the fusion protein is non-covalently linked to the cargo molecule.

14. The complex according to claim 13, wherein the fusion protein is the fusion protein according to claim 9, and the cargo molecule comprises a domain capable of specifically binding to the specific binding sequence in the fusion protein;
preferably, the domain capable of specifically binding to the specific binding sequence in the fusion protein is an amino acid sequence;
preferably, the cargo molecule is a peptide or protein;
preferably, the specific binding sequence in the fusion protein comprises a leucine zipper peptide, and the cargo molecule comprises the complementary peptide of the leucine zipper, so that the leucine zipper peptide and the complementary peptide are able to form a heterodimer;
preferably, the specific binding sequence in the fusion protein comprises leucine zipper NZ (for example, a sequence shown in SEQ ID NO: 49), and the cargo molecule comprises leucine zipper CZ (for example, a sequence shown in SEQ ID NO: 50);
preferably, the specific binding sequence in the fusion protein comprises leucine zipper CZ (for example, a sequence shown in SEQ ID NO: 50), and the cargo molecule comprises leucine zipper NZ (for example, a sequence shown in SEQ ID NO: 49).

15. The complex according to claim 13, wherein the fusion protein and the cargo molecule are conjugated by electrostatic interaction.

16. A composition, which comprises the fusion protein according to any one of claims 1 to 9, and a cargo molecule;
preferably, the cargo molecule is selected from the group consisting of nucleic acid, peptide or protein, carbohydrate, lipid, chemical compound and any mixture thereof;
preferably, the cargo molecule is selected from the group consisting of nucleic acid, peptide or protein;
preferably, the nucleic acid is selected from the group consisting of DNA molecule, RNA molecule, siRNA, antisense oligonucleotide, ribozyme, aptamer and any combination thereof.

17. The composition according to claim 16, which comprises the fusion protein according to claim 9, and the cargo molecule comprises a domain capable of specifically binding to the specific binding sequence in the fusion protein;
preferably, the domain capable of specifically binding to the specific binding sequence in the fusion protein is an amino acid sequence;
preferably, the cargo molecule is a peptide or protein;
preferably, the specific binding sequence in the fusion protein comprises a leucine zipper peptide, and the cargo molecule comprises the complementary peptide of the leucine zipper, so that the leucine zipper peptide and the complementary peptide are able to form a heterodimer;
preferably, the specific binding sequence in the fusion protein comprises leucine zipper NZ (for example, a sequence shown in SEQ ID NO: 49), and the cargo molecule comprises leucine zipper CZ (for example, a sequence shown in SEQ ID NO: 50);
preferably, the specific binding sequence in the fusion protein comprises leucine zipper CZ (for example, a sequence shown in SEQ ID NO: 50), and the cargo molecule comprises leucine zipper NZ (for example, a sequence shown in SEQ ID NO: 49).

18. An isolated nucleic acid molecule, which comprises a nucleotide sequence encoding the fusion protein according to any one of claims 1 to 9, or the complex according to claim 11, or the composition according to claim 16 or 17.

19. A vector, which comprises the isolated nucleic acid molecule according to claim 18.

20. A host cell, which comprises the isolated nucleic acid molecule according to claim 18 or the vector according to claim 19.

21. A method for preparing the fusion protein according to any one of claims 1 to 9, or the complex according to claim 11, which comprises culturing the host cell according to claim 20 under a suitable condition, and recovering the fusion protein or complex from a culture of the cell.

22. A pharmaceutical composition, which comprises the fusion protein according to any one of claims 1 to 9, the complex according to any one of claims 10 to 15, the composition according to claim 16 or 17, the isolated nucleic acid molecule according to claim 18, the vector according to claim 19 or the host cell according to claim 20, and a pharmaceutically acceptable carrier and/or excipient;
preferably, the pharmaceutical composition comprises the complex according to any one of claims 10 to 15, wherein the cargo molecule is a pharmaceutically active agent or a detectable label.

23. Use of the fusion protein according to any one of claims 1 to 9, or an isolated nucleic acid molecule, vector or host cell comprising a nucleotide sequence encoding the fusion protein, in the manufacture of a medicament.

24. Use of the complex according to any one of claims 10 to 15 or the composition according to claim 16 or 17, or an isolated nucleic acid molecule, vector or host cell comprising a nucleotide sequence encoding the complex or composition, in the manufacture of a medicament for treatment of a disease; wherein the cargo molecule contained in the complex or composition is capable of treating the disease;
preferably, the disease is a disease related to programmed cell necrosis, and the cargo molecule comprises protein phosphatase 1B; preferably, the disease related to programmed cell necrosis comprises liver injury (e.g., drug-induced liver injury), inflammatory disease, ischemia-reperfusion injury and/or neurodegenerative disease.

25. A kit, which comprises the fusion protein according to any one of claims 1 to 9, the complex according to any one of claims 10 to 15, the composition according to claim 16 or 17, the isolated nucleic acid molecule according to claim 18, the vector according to claim 19, or the host cell according to claim 20;
preferably, the kit further comprises an instruction for transfection and/or intracellular delivery.

26. Use of the fusion protein according to any one of claims 1 to 9, the complex according to any one of claims 10 to 15, the composition according to claim 16 or 17, the isolated nucleic acid molecule according to claim 18, the vector according to claim 19, or the host cell according to claim 20, as a delivery agent.

27. A method for delivering a cargo molecule into a cell, which comprises contacting the cell with the complex according to any one of claims 10 to 15, wherein the complex comprises the cargo molecule;
preferably, the contacting the cell with the complex is carried out in vitro;
preferably, the cargo molecule is selected from the group consisting of nucleic acid, peptide or protein, carbohydrate, lipid, chemical compound, and any mixture thereof; preferably, the nucleic acid is selected from the group consisting of DNA molecule, RNA molecule, siRNA, and antisense oligonucleotide, ribozyme, aptamer and any combination thereof.
